# EUROPEAN PATENT APPLICATION

(11) **EP 1 400 532 A1**
(43) Date of publication of application: **24.03.2004**
(21) Application number: 02090337.3
(22) Date of filing: 18.09.2002
(51) Int. Cl.: C07K 14/35, C07K 16/12, A61K 39/04, C12N 15/31, C12N 1/21, G01N 33/53

(54) **M. paratuberculosis specific ABC-transporter operon**

(71) Applicant: Gerlach, Gerald-F., Prof. Dr., 30449 Hannover (DE)
(72) Inventor: Gerlach, Gerald-F., Prof. Dr., 30449 Hannover (DE)
(74) Representative: Müller, Wolfram Hubertus, Dipl.-Phys.

(57) **Abstract**

The invention relates to a *Mycobacterium paratuberculosis* specific ABC-transporter operon according to claim 1, to the proteins encoded thereby as well as to a vaccine against paratuberculosis or Crohn's disease and to the development of therapeutic and/or diagnostic peptides as well as antibodies to *M. paratuberculosis*.

The present invention provides a *M. paratuberculosis* specific ABC-transporter operon which encodes several proteins useful for the induction of an immune response in a living body. The invention furthermore provides antibodies as well as therapeutic and diagnostic peptides specific to *M*. *paratuberculosis*.

SEQUENCE LISTING

<110>  Gerlach, Gerald-F.

<120>  M. paratuberculosis specific ABC-Transporter operon

<130>  GRL103

<160>  6

<170>  PatentIn version 3.1

<210>  1
<211>  8752
<212>  DNA
<213>  Mycobacterium paratuberculosis

<400>  1

gatccatgag gttcatgacg aagttctccc aggcgctcaa caggtcgccg aaaaaacctc      60

cgccgccgga agagtcgccg gtgtctcccg ccgatgagtc ctcgcttttc acaattgccg     120

gcgctgcgct gctgcgtggc gccgccgcca atgtgctggt ggagacggtc tgatacgtac     180

tcatcgtggt ggcggcctgg acccacattc gtgcgtagtc ggcctcgttg agcgcgatcg     240

ggatcgtgtt gatcccgaag aagttggttg ccaccagcgc cgcgtgagta gcgtggttcg     300

tcgctagctc gggcaaggtg ggcatggtgg ccagtgcgac tgtgtaggcg ctggccgcaa     360

tctcgtgctg ggcagcctcc cccgcggcgt cggcactgga ctggtccaac cacgctagat     420

agggagcatg tgcggccacg tacgcctcgg tggtcgggcc ctcccacgga ccggcttgga     480

ccgccgcagc gtcgcggtga gttcgtctgc ggccgaggcg tattcagcgc tcagcgacga     540

ccaagcggcg gccgcttcca gcagtggccc cggcccgggt ccgctgctca acagtgccga     600

gtgaatctct ggcggcgaag ccatccacac cggggctgtc atcgcccacc acccttggca     660

gaggacgaca ggactgctgc atcagcggtc gcatcgagaa ccgccattgc ggtggcgagc     720

ggcgacgcca tcgcgcgcgg ttggcgctga acttgtgaga ttcgacgtga tatgggcacg     780

ggctgatccc ctttcatcac atgcaaacac ccccgtaatg aaaatcattt tcattacgga     840

cggggaccct acggcgctga gacctttaat gaaagtcatt ttcaataacc cagatgtgac     900

ggaggtcaca ctggtcgcgg ggtgcgcgcc gtggcgctcg gcgtccgatg cgcgcttgac     960

cgctgatcga atgccctgcc cgaggtttgg ccgcgggggc tgcgatgcct tgcggtcgtg    1020

gcaatgctcg ggattccaca gccttcacgc cggtacccct tatgcgcgcg cgccgccaga    1080

gcgcccggag gcgaattccg ctgcgcgccg gagttttcag ctctctgggc gcaggcacag    1140

ccatcggatt aatcggcgtt cgccgcgcgg gctcgcccta ctgagcgtgt cggaagggac    1200

ggctccctac ccactgatcc atcccttgcg tggaggtatg ttatcgataa cgattttcat    1260

tatcggttca cgatggctcg gtttgagcgc ggaaggattg agcatggacc cgaatacgcc    1320

atcacaagtt gggccgcaat ccccggtttt cggcgctgcc cgcgccacgc tcattggatg    1380

cgcgctgttg accgcactca gcgcccttgc cgggatggca ccgctgatcg cggtggtgga    1440

ggtcagccga cggctactag ctggtgacac tgctgtttgg gcgatcattg cagcggcgct    1500

cgttgcgctc gccgtcaagc agcttggtac gttttgcgct ggcgttctga cccacctggc    1560

tgacatccag gtctcgtttc gaattcgacg agatttgctc gtgaagctga gcaaactgcc 1620
actcggctgg tttaccgacc gcaactccgg gattgtcaag aagaccgttg aagacgacgt 1680
cagcggccct gcaccagctg atcgcccatt ccatcgtgga actcaccgcg gcgacagttc 1740
ccccattagt cgccttgacc tacctgtttg ctgttgactg gcgcatggcg ctcgtcactt 1800
tgctcccggt actgattggg ctggtcatgt atcagcgcgc gatggccagc gcggacacca 1860
agtatccaga gttcatgcag tggctggccc gactcaacag cgctgcggtg gagttcgtga 1920
acgggatcgg cgtcgtcaaa gcgttcggca cgccaggtgt ggcgagccgc cgctttcagg 1980
aagtgtcgcg tgcctttgcg cacttttttc tcgactgggc caaatccacc agcgtcgcag 2040
cggttatcgc cgaaacgcta ctgtcgccgc ccagcattct ggttgtcgtc gcgggggccg 2100
gcggagctct gaccgccaac gggaagctgc cgctggatag ctttatcgcg ttcctggttt 2160
tcgggactgt gataaccgcc ggtctgatga ccgtgatgat gtcgatacac ccgctggtca 2220
ctgccctggg agtcgcacgc gatatccgcg aactgctggc cggcccggag ctgccgacac 2280
cggatgagcc ggtcggtctc gatccatcca gcgccgcgcc ggtggtgcgc ctgcgtgacg 2340
tggtgttcac ctacggtgag accgttgcgc tgtccggcat caacctcgaa ctcgaccgca 2400
acaccattac agcgttggtc ggcccttccg gatccgggaa atcgaccctc gccaagttac 2460
tgccccggtt cgacgatccg gtcgaaggtt cggttgagct ttacggtgtg gacctacgca 2520
gcgtcgaccc cgccgagttg tatcgacatg tcgctttcgt gtttcaagac gccgaactgt 2580
tacgcatgag catccgcgac aacatccgac tcagccggcc ggacgccgac gacgtcgcgg 2640
tccgcgacgc cgcggacagg gcccaaatcc tagagcggat cgagcaattg ccgcgcggct 2700
ttgattcggt tgtgggcgag gacgccgagc tttccggggg cgaacggcag cgcgtctgca 2760
tcgcccgcgc catcttggcc gaccgccccg tgctggtcct cgacgaggcc acggccagcg 2820
ccgatcctga aaacgaagct cgaatccagg acgcgctcaa cgaagccgct tcgggccgga 2880
cggtattggt aatcgcccat cgcctcaaca ccattcgtgg cgctgaccac atcgtcgtac 2940
tcgacgacgg ccgaatcgcc gaacaaggca cccatttcga gcttttggag agcaaaggac 3000
tttatgcgcg tttgtgggag catgaccgtc gtgttcagag cggcgtcgcc gaacacatgg 3060
aggttgggcg atgagcacga tgattcgtta tttgcttgaa ttacttgacg aacgcggtcg 3120
gcgtcacctt cgcgtgatgc ttgccttgca agccgcacag ggaattctgc agggactcgg 3180
tttccttttc gtcgtaccgc tgatgggtgt gctcatccac aagccggttg atacgcacgc 3240
cctttggtgc tggattgtgg cgatcgccgt cgctgtcgtc gctcaccacg gactgcttgc 3300
gtggagcacc tcgctgggtt acctggtcgg cacagatgtg ctgaccagct ttcacacccg 3360
catcggcaac catctcgcca cgctgccaat cggctggttc cgcaccgatc gcaccggacc 3420
gatcgggcgt ctgctcagca aggggaccat ggacgtcgcg gaccttcccg cgcacctgct 3480
gcggcatgtc atcgtgggtg tagccgcgcc tgccaccatg atcatcggaa gctatgtgct 3540
ggactggcgg gtcgggctcg cattcaccgc cggcgcgctg ctatgcgcac tgacgcttcg 3600
actactcatg gtcgtcatcc accgcaacga cgaccagtac gatcacgaca tcggagtgac 3660
cgcgtcacgc atcgtcgagt acgcccgtca gcagccgaca ctgcgtgcat acggcgtcct 3720

gaaccggccc ggactgggaa cgctagagga atcgcttgtc cggcaacaga cttcgcagtc 3780
ccgcttgacc attcgcggcg cgtgggcgct catcggattc ttcggaaccg tccaactggt 3840
ggtgaccgcc atcatcgcgc tcaccgtcgc tctcgcagta agcggcagcc ttgcgctttc 3900
gacgatggtc ggtttgctga tcatcacctt gcgcatgatc gacccgattt ctcagctcgg 3960
cgatctcgcc ggccatgtcc aggtcaacgc cgacgccatc cgccgagtgc gtgaactgtt 4020
gacggtaccc ccgttgcccg aacccaccca tccaggtcaa gccacgggtg ccgacatcga 4080
attgcagggg gttcgtttcg cttacgacgg aggtcaaccc accatcgatg gtatcgacgc 4140
cgtctttcca caaggtagcc tgactgccgt cgtcggcccg tctggcgcgg gaaaaagcac 4200
gctgctcaaa ctaatcgggc ggttcttcga cgtcgacgaa ggcagcatca ccatcggcgg 4260
ctgcgacatc cgccaactcg gcaccgccgg cgtctcacat ctcacggcgc aagttttcca 4320
agacgtgtac ctctttgaag gcacaattgc cgacaacctg cggatggccg atccgcaagc 4380
caccgatgat gatctccgcc acgtcgccac gatcagtcgc ctcgacgagg tagtcgatcg 4440
ccttcccaat gggtgggaaa cccgcgttgg tgaggcgggg tcgacgctgt ccggcgggga 4500
aaagcagcgt gtctccatag cgcgcgccct gctcaaagac gcaccggtag tactgctgga 4560
cgaggcgacc gcggcgctgg atgccgccaa tgagaccgcc gtttccgacg ctatccacga 4620
actagcgcgt gaccgcacag tcatcgttgt cgcacaccgc ctttcgaccg tggcggccgc 4680
cgatcagata ctggtgctcg acggcggccg gatcaccgag cgtggccgcc acgacacact 4740
ggtgggtgcg ggcggcacct acgcccgctt ctgggaagaa cgaactcgcg cgcgagggtg 4800
gcaactgcgc caaattgcgc ccactcctga actgcaggca cctcgaccat gacggccact 4860
agctcgacga cccagtccag tcgccgcatc gacgtccgga tgagcgccag ggatctcatc 4920
aacatcgggg tcttcggcgc tctctacatc gccactgtgt tcgcgatcaa cgtgttcgct 4980
ttcatcaatc cgctcgtcat gttggtcgcc ctggcggtca gcatgatcgc cggcggcgtg 5040
ccgttcatgt tgttcctcac ccgggtgcga catgcgggca tggtgacggt gtttgcgatt 5100
atcacggccg gactgctcgc actgaccggg caccccccga tctgcttcgt gatcacagtt 5160
gcgtgcgcgt tggtggccga agtcgtcctg tggctgggac gctatcgctc ccgcaccatg 5220
ggtgtactgg cgtacgcaat ctacgcggcg tggtacatcg ggccgctgct gcccatcttc 5280
tacgctcgcg atgaatattt ctccagtccc ggcatggcac agatgggtcc gcgctacctc 5340
gaagagatgg aacggttgtt gtcgccagcc gtgctaatcg cattcgacct gtccacggtg 5400
gtattcgggc tgatcggcgg actgctcgga gtaaggttgc tgcgcaagca ttttcagagg 5460
gccggcctag cttgacgcgg acaacgactc gggtgaccca actcgaccct cgaaccaaaa 5520
ccgtgctggt gctggcctca agcatcgccg tgatggcccc cggtggcgag gtgttcgtcc 5580
cagccgccgt catcgttggg atgctgctgg cggttgccga gcaggcgtgg gtgcgggctg 5640
ccattcttcc ctcggctgcc ggggccacgg ccgcagtcgc ttacctacta ccacaagcta 5700
ttccacatcc gataatcggc gcaatcggta ccgtagccgc ctacctgcta cggctcatcg 5760
ccgtcggcgc aatcgtcatt catctggtga acaccacgac accgagcgaa ttcaccgctg 5820

ccctgcgcgc tacccacatt ccccgtgcga tcacggtctc cggttccgtc atgctgcgat 5880
tcctgccaac catcgttggt gaagcacgtg ccgtcagcga cgccatgcga ttgcgtggga 5940
tcggcgggac atacggaatg ctgcgccatc ccgtttgcac catcgagtac ttcaccgttc 6000
cccttatagc gtcgagcctt cgtgtcgcag aagatctttc agcaaccgcg ctactgcgtg 6060
gtcttggctc tgcagcacgg ccgacaacga tgtacccccc gcgtttcggt aaagcggatg 6120
cgctaatcgg ttgcatcgtc agcgcactga ccgtcaccac cgtgctgtgg ccggtgaaac 6180
cgtgattcgc atcgacggcg tacgttggca atacgccgga accgacgctg ccgttttgga 6240
cggagtggac ttgcacatac gccgtggcga gaccgttctg ctctgcgggg ccagcggatc 6300
gggcaagagc agcgtcctgc ggctgatgaa tggccttatc ccacatttcc accagggcag 6360
cctcgacggt agcgtacaca ttgacggtac atcggtcgcc gaattgtctc tggaaagggt 6420
tggtcggctg acaggtaccg tattgcaaca cccgcgccga caattcttca ccgcggctgt 6480
cgacaccgaa ttggctttca ctctggaaaa tttcgggaca ccgcccgagc agatccgaaa 6540
ccgtgtcggc tccgtgatca ccgaatacgg gctcgcagaa ctcaccggcc accgattggc 6600
ggagttgtcc ggcggccagc agcaacagat cgcctgcgcg gccgccgcca cccacggtcc 6660
gccgctgctg ttgttcgacg agccaacggc aaacctggct gcggacgcga tcgagcgttt 6720
cacagcaacg cttgcgaggc ttcgctcact gggaaccacg atcgtcattg ccgaacaccg 6780
gctgcactac ctacgcgaaa tcgcggaccg aatcgtcctg ctacgcaacg gacgtatcgc 6840
cgccgaatgg tcgcgtaagc aattcgcgcg tcttgacgat gcggcgttga acgcagaggg 6900
cctgcgcagc aacaattctc cagtgcgcaa tcacataccc ccggcgtgcg catacggcgc 6960
aagcgttgcg ggcacaccat cgggcacggc tgccccagct tcgtcaccgt cggaggtggt 7020
gctgcgaggt attcgttgtt gcttccgcgg ccaccgcgta ctcgacatcg aagaggcgcg 7080
gtttccggct gcaacggtca ccgccatcac cggcccgaac ggcgcgggca aaagtaccct 7140
ggccagggtt ctcgtcgggc tgcaacgtca tgacggcgag gtgtcgtttg gcggaagccg 7200
gatatctcac agccgtcggc agcgaatgtc accgatcgtg atgcaggatg tgcagcggca 7260
gctgttcacc gagtctgtcc gtgccgaact acggctcggt gcacccccgg cggcagcagg 7320
tgttgcatcc accctgttaa gagacctggg gctggaagag ttcgccgatc ggcacccatt 7380
gtcactttcg ggaggacagc agcaacgtct ggtggtcgcc gccgcacggc tgagcaatcg 7440
caagatcatg gttttcgacg aaccgagctc gggtgtcgac cggcgccacc tccgctcgat 7500
caccaatgtc atgcgtgacg tcgcggccca aggagtagtc gttatcttga tcagtcacga 7560
tcaggagctg ttgactctcg cggctgacca ggaactgcgc atgcgggttg ccgacactct 7620
caatgcgaga tcccgtagga aggcggccgg cgaaaatgct tgccttgaaa cgctatccga 7680
ctagcagacc cccgctcgca tgttacagcg acaaagcact cagattgttc tgagctacaa 7740
cagtgaccgg gtgcgcggga agtctgttgg agatcgtctg ccttgcccgg gtcgccccct 7800
atggatgcgt taccgtcaac gcagacgtcg agccagcagc acatcgtcgt gcgtgtgctg 7860
cagttctggc ctggcggctg tcttgcggcg ctgccgtctg tcgacccgga catgccaatc 7920
gccgtcaagc aacgtggcaa tgtcgtcatg gctgaggtag tcggctgggt cgaacccgta 7980

ggattttgcc ttttccgcgt caacgtcggc atggtgtacg acaaggagtg tccctccggg 8040

agcgacagct gcaagcaggg actcctcagc ggcgcgcccc gacgagtgac gcaacggccc 8100

ggataaaacg caataaccag gtcgtagccg ccaactcgaa gcggtgcgtt ttcgaatgcg 8160

gcaccgcacc cagttaaccc tgaccccttg cttgtcgacc gggagcccgt tgcgcgtgct 8220

taatgcgaac tcgtgacacg tccaacgccg tcacgtccca ttcacgactg gccagccaaa 8280

tcgcttcagc gcctacgccg cacccgatgt cgagtgccct tcccggcgcg aacttgctta 8340

cctcagttat cagcacggca ttgggttctg catcgcgcat ctctgtcgtc aaaccggcgt 8400

agcgctcatc ccactcagca gccagggcgt tggtgtcgtc cggtgaagct gtcatgcata 8460

ccagcatggc ccaggtaccg aagacccaca atcgcgatcg ccaaatcggc aaactgcctc 8520

gacatcgctc ggcaagttcg atcgttcagc aaagtcgctt tcgggcggga catccgaatg 8580

tcaacaactt ttgaaagctg accccgtacc aacttctgaa tcttgccccc gggtgcggtc 8640

ctgcccgcga gagcatttgc tttggtgacc gtcgctctgc tctgcgggca gactcgatac 8700

ccgcaacccc taacggtcag atccgttgtg cggcacgcat aaccgattga tc 8752

<210> 2
<211> 340
<212> PRT
<213> Mycobacterium paratuberculosis

<400> 2

Val Arg Phe Asp Val Ile Trp Ala Arg Ala Asp Pro Leu Ser Ser His
1               5                   10                  15

Ala Asn Thr Pro Val Met Lys Ile Ile Phe Ile Thr Asp Gly Asp Pro
            20                  25                  30

Thr Ala Leu Arg Pro Leu Met Lys Val Ile Phe Asn Asn Pro Asp Val
        35                  40                  45

Thr Glu Val Thr Leu Val Ala Gly Cys Ala Pro Trp Arg Ser Ala Ser
    50                  55                  60

Asp Ala Arg Leu Thr Ala Asp Arg Met Pro Cys Pro Arg Phe Gly Arg
65                  70                  75                  80

Gly Gly Cys Asp Ala Leu Arg Ser Trp Gln Cys Ser Gly Phe His Ser
                85                  90                  95

Leu His Ala Gly Thr Pro Tyr Ala Arg Ala Pro Pro Glu Arg Pro Glu
            100                 105                 110

Ala Asn Ser Ala Ala Arg Arg Ser Phe Gln Leu Ser Gly Arg Arg His
        115                 120                 125

Ser His Arg Ile Asn Arg Arg Ser Pro Arg Gly Leu Ala Leu Leu Ser
    130                 135                 140

Val Ser Glu Gly Thr Ala Pro Tyr Pro Leu Ile His Pro Leu Arg Gly
145                 150                 155                 160

Gly Met Leu Ser Ile Thr Ile Phe Ile Ile Gly Ser Arg Trp Leu Gly
                165                 170                 175

Leu Ser Ala Glu Gly Leu Ser Met Asp Pro Asn Thr Pro Ser Gln Val
            180                 185                 190

Gly Pro Gln Ser Pro Val Phe Gly Ala Ala Arg Ala Thr Leu Ile Gly
        195                 200                 205

Cys Ala Leu Leu Thr Ala Leu Ser Ala Leu Ala Gly Met Ala Pro Leu
    210                 215                 220

Ile Ala Val Val Glu Val Ser Arg Arg Leu Leu Ala Gly Asp Thr Ala
225                 230                 235                 240

Val Trp Ala Ile Ile Ala Ala Ala Leu Val Ala Leu Ala Val Lys Gln
                245                 250                 255

Leu Gly Thr Phe Cys Ala Gly Val Leu Thr His Leu Ala Asp Ile Gln
            260                 265                 270

Val Ser Phe Arg Ile Arg Arg Asp Leu Leu Val Lys Leu Ser Lys Leu
        275                 280                 285

Pro Leu Gly Trp Phe Thr Asp Arg Asn Ser Gly Ile Val Lys Lys Thr
    290                 295                 300

Val Glu Asp Asp Val Ser Gly Pro Ala Pro Ala Asp Arg Pro Phe His
305                 310                 315                 320

Arg Gly Thr His Arg Gly Asp Ser Ser Pro Ile Ser Arg Leu Asp Leu
                325                 330                 335

Pro Val Cys Cys
            340

<210>  3
<211>  593
<212>  PRT
<213>  Mycobacterium paratuberculosis

<400>  3

Met Ser Thr Met Ile Arg Tyr Leu Leu Glu Leu Leu Asp Glu Arg Gly
1               5                   10                  15

Arg Arg His Leu Arg Val Met Leu Ala Leu Gln Ala Ala Gln Gly Ile
            20                  25                  30

Leu Gln Gly Leu Gly Phe Leu Phe Val Val Pro Leu Met Gly Val Leu
        35                  40                  45

Ile His Lys Pro Val Asp Thr His Ala Leu Trp Cys Trp Ile Val Ala
    50                  55                  60

Ile Ala Val Ala Val Val Ala His His Gly Leu Leu Ala Trp Ser Thr
65                  70                  75                  80

Ser Leu Gly Tyr Leu Val Gly Thr Asp Val Leu Thr Ser Phe His Thr
                85                  90                  95

Arg Ile Gly Asn His Leu Ala Thr Leu Pro Ile Gly Trp Phe Arg Thr
            100                 105                 110

Asp Arg Thr Gly Pro Ile Gly Arg Leu Leu Ser Lys Gly Thr Met Asp
        115                 120                 125

Val Ala Asp Leu Pro Ala His Leu Leu Arg His Val Ile Val Gly Val
    130                 135                 140

Ala Ala Pro Ala Thr Met Ile Ile Gly Ser Tyr Val Leu Asp Trp Arg
145                 150                 155                 160

Val Gly Leu Ala Phe Thr Ala Gly Ala Leu Leu Cys Ala Leu Thr Leu
                165                 170                 175

Arg Leu Leu Met Val Val Ile His Arg Asn Asp Asp Gln Tyr Asp His
            180                 185                 190

Asp Ile Gly Val Thr Ala Ser Arg Ile Val Glu Tyr Ala Arg Gln Gln
        195                 200                 205

Pro Thr Leu Arg Ala Tyr Gly Val Leu Asn Arg Pro Gly Leu Gly Thr
    210                 215                 220

Leu Glu Glu Ser Leu Val Arg Gln Gln Thr Ser Gln Ser Arg Leu Thr
225                 230                 235                 240

Ile Arg Gly Ala Trp Ala Leu Ile Gly Phe Phe Gly Thr Val Gln Leu
                245                 250                 255

Val Val Thr Ala Ile Ile Ala Leu Thr Val Ala Leu Ala Val Ser Gly
            260                 265                 270

Ser Leu Ala Leu Ser Thr Met Val Gly Leu Leu Ile Ile Thr Leu Arg
        275                 280                 285

Met Ile Asp Pro Ile Ser Gln Leu Gly Asp Leu Ala Gly His Val Gln
    290                 295                 300

Val Asn Ala Asp Ala Ile Arg Arg Val Arg Glu Leu Leu Thr Val Pro
305                 310                 315                 320

Pro Leu Pro Glu Pro Thr His Pro Gly Gln Ala Thr Gly Ala Asp Ile
                325                 330                 335

Glu Leu Gln Gly Val Arg Phe Ala Tyr Asp Gly Gly Gln Pro Thr Ile
            340                 345                 350

Asp Gly Ile Asp Ala Val Phe Pro Gln Gly Ser Leu Thr Ala Val Val
        355                 360                 365

Gly Pro Ser Gly Ala Gly Lys Ser Thr Leu Leu Lys Leu Ile Gly Arg
    370                 375                 380

Phe Phe Asp Val Asp Glu Gly Ser Ile Thr Ile Gly Gly Cys Asp Ile
385                 390                 395                 400

Arg Gln Leu Gly Thr Ala Gly Val Ser His Leu Thr Ala Gln Val Phe
                405                 410                 415

Gln Asp Val Tyr Leu Phe Glu Gly Thr Ile Ala Asp Asn Leu Arg Met
            420                 425                 430

Ala Asp Pro Gln Ala Thr Asp Asp Asp Leu Arg His Val Ala Thr Ile
        435                 440                 445

Ser Arg Leu Asp Glu Val Val Asp Arg Leu Pro Asn Gly Trp Glu Thr
    450                 455                 460

Arg Val Gly Glu Ala Gly Ser Thr Leu Ser Gly Gly Glu Lys Gln Arg
465                 470                 475                 480

Val Ser Ile Ala Arg Ala Leu Leu Lys Asp Ala Pro Val Val Leu Leu
                485                 490                 495

Asp Glu Ala Thr Ala Ala Leu Asp Ala Ala Asn Glu Thr Ala Val Ser
            500                 505                 510

Asp Ala Ile His Glu Leu Ala Arg Asp Arg Thr Val Ile Val Val Ala
        515                 520                 525

His Arg Leu Ser Thr Val Ala Ala Ala Asp Gln Ile Leu Val Leu Asp
    530                 535                 540

Gly Gly Arg Ile Thr Glu Arg Gly Arg His Asp Thr Leu Val Gly Ala
545                 550                 555                 560

Gly Gly Thr Tyr Ala Arg Phe Trp Glu Glu Arg Thr Arg Ala Arg Gly
                565                 570                 575

Trp Gln Leu Arg Gln Ile Ala Pro Thr Pro Glu Leu Gln Ala Pro Arg
            580                 585                 590

Pro

<210> 4
<211> 208

<212>  PRT
<213>  Mycobacterium paratuberculosis

<400>  4

Met Thr Ala Thr Ser Ser Thr Thr Gln Ser Ser Arg Arg Ile Asp Val
1               5                   10                  15

Arg Met Ser Ala Arg Asp Leu Ile Asn Ile Gly Val Phe Gly Ala Leu
            20                  25                  30

Tyr Ile Ala Thr Val Phe Ala Ile Asn Val Phe Ala Phe Ile Asn Pro
        35                  40                  45

Leu Val Met Leu Val Ala Leu Ala Val Ser Met Ile Ala Gly Gly Val
    50                  55                  60

Pro Phe Met Leu Phe Leu Thr Arg Val Arg His Ala Gly Met Val Thr
65                  70                  75                  80

Val Phe Ala Ile Ile Thr Ala Gly Leu Leu Ala Leu Thr Gly His Pro
                85                  90                  95

Pro Ile Cys Phe Val Ile Thr Val Ala Cys Ala Leu Val Ala Glu Val
            100                 105                 110

Val Leu Trp Leu Gly Arg Tyr Arg Ser Arg Thr Met Gly Val Leu Ala
        115                 120                 125

Tyr Ala Ile Tyr Ala Ala Trp Tyr Ile Gly Pro Leu Leu Pro Ile Phe
    130                 135                 140

Tyr Ala Arg Asp Glu Tyr Phe Ser Ser Pro Gly Met Ala Gln Met Gly
145                 150                 155                 160

Pro Arg Tyr Leu Glu Glu Met Glu Arg Leu Leu Ser Pro Ala Val Leu
                165                 170                 175

Ile Ala Phe Asp Leu Ser Thr Val Val Phe Gly Leu Ile Gly Gly Leu
            180                 185                 190

Leu Gly Val Arg Leu Leu Arg Lys His Phe Gln Arg Ala Gly Leu Ala
        195                 200                 205

<210>  5
<211>  210
<212>  PRT
<213>  Mycobacterium paratuberculosis

<400>  5

Met Ala Pro Gly Gly Glu Val Phe Val Pro Ala Ala Val Ile Val Gly
1               5                   10                  15

Met Leu Leu Ala Val Ala Glu Gln Ala Trp Val Arg Ala Ala Ile Leu
            20                  25                  30

Pro Ser Ala Ala Gly Ala Thr Ala Ala Val Ala Tyr Leu Leu Pro Gln
        35                  40                  45

Ala Ile Pro His Pro Ile Ile Gly Ala Ile Gly Thr Val Ala Ala Tyr
    50                  55                  60

Leu Leu Arg Leu Ile Ala Val Gly Ala Ile Val Ile His Leu Val Asn
65                  70                  75                  80

Thr Thr Thr Pro Ser Glu Phe Thr Ala Ala Leu Arg Ala Thr His Ile
                85                  90                  95

Pro Arg Ala Ile Thr Val Ser Gly Ser Val Met Leu Arg Phe Leu Pro
            100                 105                 110

Thr Ile Val Gly Glu Ala Arg Ala Val Ser Asp Ala Met Arg Leu Arg
        115                 120                 125

Gly Ile Gly Gly Thr Tyr Gly Met Leu Arg His Pro Val Cys Thr Ile
    130                 135                 140

Glu Tyr Phe Thr Val Pro Leu Ile Ala Ser Ser Leu Arg Val Ala Glu
145                 150                 155                 160

Asp Leu Ser Ala Thr Ala Leu Leu Arg Gly Leu Gly Ser Ala Ala Arg
                165                 170                 175

Pro Thr Thr Met Tyr Pro Pro Arg Phe Gly Lys Ala Asp Ala Leu Ile
            180                 185                 190

Gly Cys Ile Val Ser Ala Leu Thr Val Thr Thr Val Leu Trp Pro Val
        195                 200                 205

Lys Pro
    210

<210> 6
<211> 500
<212> PRT
<213> Mycobacterium paratuberculosis

<400> 6

Val Ile Arg Ile Asp Gly Val Arg Trp Gln Tyr Ala Gly Thr Asp Ala
1               5                   10                  15

Ala Val Leu Asp Gly Val Asp Leu His Ile Arg Arg Gly Glu Thr Val
            20                  25                  30

Leu Leu Cys Gly Ala Ser Gly Ser Gly Lys Ser Ser Val Leu Arg Leu
        35                  40                  45

Met Asn Gly Leu Ile Pro His Phe His Gln Gly Ser Leu Asp Gly Ser
    50                  55                  60

Val His Ile Asp Gly Thr Ser Val Ala Glu Leu Ser Leu Glu Arg Val
65                  70                  75                  80

Gly Arg Leu Thr Gly Thr Val Leu Gln His Pro Arg Arg Gln Phe Phe
                85                  90                  95

Thr Ala Ala Val Asp Thr Glu Leu Ala Phe Thr Leu Glu Asn Phe Gly
            100                 105                 110

Thr Pro Pro Glu Gln Ile Arg Asn Arg Val Gly Ser Val Ile Thr Glu
        115                 120                 125

Tyr Gly Leu Ala Glu Leu Thr Gly His Arg Leu Ala Glu Leu Ser Gly
    130                 135                 140

Gly Gln Gln Gln Gln Ile Ala Cys Ala Ala Ala Ala Thr His Gly Pro
145                 150                 155                 160

Pro Leu Leu Leu Phe Asp Glu Pro Thr Ala Asn Leu Ala Ala Asp Ala
                165                 170                 175

Ile Glu Arg Phe Thr Ala Thr Leu Ala Arg Leu Arg Ser Leu Gly Thr
            180                 185                 190

Thr Ile Val Ile Ala Glu His Arg Leu His Tyr Leu Arg Glu Ile Ala
        195                 200                 205

Asp Arg Ile Val Leu Leu Arg Asn Gly Arg Ile Ala Ala Glu Trp Ser
    210                 215                 220

Arg Lys Gln Phe Ala Arg Leu Asp Asp Ala Ala Leu Asn Ala Glu Gly
225                 230                 235                 240

Leu Arg Ser Asn Asn Ser Pro Val Arg Asn His Ile Pro Pro Ala Cys
                245                 250                 255

Ala Tyr Gly Ala Ser Val Ala Gly Thr Pro Ser Gly Thr Ala Ala Pro
            260                 265                 270

Ala Ser Ser Pro Ser Glu Val Val Leu Arg Gly Ile Arg Cys Cys Phe
        275                 280                 285

Arg Gly His Arg Val Leu Asp Ile Glu Glu Ala Arg Phe Pro Ala Ala
    290                 295                 300

Thr Val Thr Ala Ile Thr Gly Pro Asn Gly Ala Gly Lys Ser Thr Leu
305                 310                 315                 320

Ala Arg Val Leu Val Gly Leu Gln Arg His Asp Gly Glu Val Ser Phe
                325                 330                 335

Gly Gly Ser Arg Ile Ser His Ser Arg Arg Gln Arg Met Ser Pro Ile
            340                 345                 350

Val Met Gln Asp Val Gln Arg Gln Leu Phe Thr Glu Ser Val Arg Ala
        355                 360                 365

Glu Leu Arg Leu Gly Ala Pro Pro Ala Ala Ala Gly Val Ala Ser Thr
    370                 375                 380

Leu Leu Arg Asp Leu Gly Leu Glu Glu Phe Ala Asp Arg His Pro Leu
385                 390                 395                 400

Ser Leu Ser Gly Gly Gln Gln Gln Arg Leu Val Val Ala Ala Ala Arg
                405                 410                 415

Leu Ser Asn Arg Lys Ile Met Val Phe Asp Glu Pro Ser Ser Gly Val
            420                 425                 430

Asp Arg Arg His Leu Arg Ser Ile Thr Asn Val Met Arg Asp Val Ala
        435                 440                 445

Ala Gln Gly Val Val Val Ile Leu Ile Ser His Asp Gln Glu Leu Leu
    450                 455                 460

Thr Leu Ala Ala Asp Gln Glu Leu Arg Met Arg Val Ala Asp Thr Leu
465                 470                 475                 480

Asn Ala Arg Ser Arg Arg Lys Ala Ala Gly Glu Asn Ala Cys Leu Glu
                485                 490                 495

Thr Leu Ser Asp
            500

## Description

The present invention relates to a *M*. *paratuberculosis* specific ABC-transporter operon, its encoded proteins und their use for the production of vaccines and/or antibodies as well as therapeutic and diagnostic peptides.

Paratuberculosis, also called Johne's disease, is a severe and incurable gastroenteritis of ruminants caused by *Mycobacterium avium* subspecies (ssp.) *paratuberculosis* (*M*. *paratuberculosis;* Kreeger, J. M. 1991. Ruminant paratuberculosis - a century of progress and frustration. J.Vet.Diagn.Invest 3:373-382). In cattle, infection most commonly occurs in newborn calves through the fecal-oral route. After a long incubation period, clinical symptoms including persistent diarrhea and weight loss predominantly develop in animals three to five years of age (Chiodini, R. J., H. J. Van Kruiningen, and R. S. Merkal. 1984. Ruminant paratuberculosis (Johne's disease): the current status and future prospects. Cornell Vet. 74:218-262). The disease is prevalent in domestic animals and wildlife worldwide and has a considerable economic impact on the livestock industry (Harris, N. B. and R. G. Barletta. 2001. Mycobacterium avium subsp. paratuberculosis in Veterinary Medicine. Clin.Microbiol.Rev. 14:489-512).

*M*. paratuberculosis is a member of the Myobycterium avium complex (MAC) additionally comprising *M*. *avium* ssp. *avium* (*M*. *avium*) and *M*. *avium* ssp. *silvaticum* (*M*. *silvaticum).* At the subspecies level, *M*. *paratuberculosis* can be differentiated phenotypically from *M*. *avium* and *M*. *silvaticum* by its dependence on the iron chelator mycobactin for growth in culture (De Voss, J. J., K. Rutter, B. G. Schroeder, and C. E. Barry, III. 1999. Iron acquisition and metabolism by mycobacteria. J.Bacteriol. 181:4443-4451; Thorel, M. F., M. Krichevsky, and V. V. Levy-Frebault. 1990. Numerical taxonomy of mycobactin-dependent mycobacteria, emended description of Mycobacterium avium, and description of Mycobacterium avium subsp. avium subsp. nov., Mycobacterium avium subsp. paratuberculosis subsp. nov., and Mycobacterium avium subsp. silvaticum subsp. nov. Int.J.Syst.Bacteriol. 40:254-260) and genotypically by the presence of multiple copies of the insertion elements IS900 (Green, E. P., M. L. Tizard, M. T. Moss, J. Thompson, D. J. Winterbourne, J. J. McFadden, and J. Hermon-Taylor. 1989. Sequence and characteristics of IS900, an insertion element identified in a human Crohn's disease isolate of Mycobacterium paratuberculosis. Nucleic Acids Res. 17:9063-9073) and ISMav2 (Strommenger, B., K. Stevenson, and G. F. Gerlach. 2001. Isolation and diagnostic potential of ISMav2, a novel insertion sequence-like element from Mycobacterium avium ssp. paratuberculosis. FEMS Microbiol.Lett. 196:31-37). The three closely related M. *avium* subspecies show distinct host preferences with M. *paratuberculosis* being pathogenic mainly for ruminants and *M*. *avium* and *M*. *silvaticum* being primarily pathogenic in birds (Thorel, M. F., M. Krichevsky, and V. V. Levy-Frebault. 1990). Despite its host preference, *M*. *paratuberculosis* has also been found in other lifestock (Grange, J. M., M. D. Yates, and E. Boughton. 1990. The avian tubercle bacillus and its relatives. J.Appl.Bacteriol. 68:411-431.; Thorel, M. F., H. Huchzermeyer, R. Weiss, and J. J. Fontaine. 1997. Mycobacterium avium infections in animals. Literature review. Vet. Res. 28:439-447), in non-ruminant wildlife (Beard, P. M., M. J. Daniels, D. Henderson, A. Pirie, K. Rudge, D. Buxton, S. Rhind, A. Greig, M. R. Hutchings, I. McKendrick, K. Stevenson, and J. M. Sharp. 2001. Paratuberculosis infection of nonruminant wildlife in Scotland. J.Clin.Microbiol. 39:1517-1521; Beard, P. M., S. M.

Rhind, D. Buxton, M. J. Daniels, D. Henderson, A. Pirie, K. Rudge, A. Greig, M. R. Hutchings, K. Stevenson, and J. M. Sharp. 2001. Natural paratuberculosis infection in rabbits in Scotland. J.Comp Pathol. 124:290-299; Greig, A., K. Stevenson, V. Perez, A. A. Pirie, J. M. Grant, and J. M. Sharp. 1997. Paratuberculosis in wild rabbits (Oryctolagus cuniculus). Vet.Rec. 140:141-143), and in intestinal tissue of Crohn's disease patients (Chiodini, R. J. 1989. Crohn's disease and the mycobacterioses: a review and comparison of two disease entities. Clin.Microbiol.Rev. 2:90-117; Hulten, K., H. M. El Zimaity, T. J. Karttunen, A. Almashhrawi, M. R. Schwartz, D. Y. Graham, and F. A. El Zaatari . 2001. Detection of Mycobacterium avium subspecies paratuberculosis in Crohn's diseased tissues by in situ hybridization. Am.J.Gastroenterol. 96:1529-1535).

This as well as the finding that *M*. *paratuberculosis* is shed with milk from infected cows, and, in contrast to other mycobacteria, survives pasteurization temperatures and cheese production (Sung, N. and M. T. Collins. 1998. Thermal tolerance of Mycobacterium paratuberculosis. Appl.Environ.Microbiol. 64:999-1005; Sung, N. and M. T. Collins. 2000. Effect of three factors in cheese production (pH, salt, and heat) on Mycobacterium avium subsp. paratuberculosis viability. Appl.Environ.Microbiol. 66:1334-1339) led to the concern that the bacterium may present a potential risk for human health. Although the relationship between *M*. *paratuberculosis* and Crohn's disease is still controversially discussed, evidence has accumulated that *M*. *paratuberculosis* is very likely a cause of Crohn's disease (Cook, L. S. 2000. A causal role for Mycobacterium avium subspecies paratuberculosis in Crohn's disease? Can.J.Gastroenterol. 14:479-480; Hermon-Taylor, J., T. J. Bull, J. M. Sheridan, J. Cheng, M. L.

Stellakis, and N. Sumar. 2000. Causation of Crohn's disease by Mycobacterium avium subspecies paratuberculosis. Can.J.Gastroenterol. 14:521-539).

The problem to be solved by the present invention is to provide a vaccine and antibodies as well as therapeutic and diagnostic peptides that are specific to *M*. *paratuberculosis.*

The present invention provides a *M*. *paratuberculosis* specific ABC-transporter operon which encodes several proteins useful for the induction of an immune response in a living body. The invention furthermore provides antibodies as well as therapeutic and diagnostic peptides specific to *M*. *paratuberculosis*.

The present invention accordingly provides a M. paratuberculosis specific ABC-transporter operon with the sequence according to SEQ ID NO 1.

According to the invention, a *M*. *paratuberculosis* specific ABC-transporter operon is preferred, that comprises six open reading frames mptA, mptB, mptC, mptD, mptE and mptF.

Furthermore, a *M*. *paratuberculosis* specific ABC-transporter operon that encodes five proteins is preferred.

Preferred is a *M*. *paratuberculosis* specific ABC-transporter operon, that encodes the proteins MptAB, MptC, MptD, MptE and MptF.

Another preferred embodiment is the protein MptAB with the sequence according to SEQ ID NO 2.

It is further preferred that MptAB is an ABC-transporter protein.

Another preferred embodiment is the protein MptC with the sequence according to SEQ ID NO 3.

It is preferred that the protein MptC has a molecular mass of 64 kilodalton.

It is particularly preferred that the MptC protein is an ABC-transporter protein.

A preferred embodiment of the invention is the protein MptD with the sequence according to SEQ ID NO 4.

It is preferred that protein MptD is a transmembrane protein.

Also preferred is the protein MptE with the sequence according to SEQ ID NO 5.

Furthermore preferred is the protein MptF with the sequence according to SEQ ID NO 6.

Preferred are transformants of *M*. *smegmatis* mc²155 containing the *M*. *paratuberculosis* specific ABC-transporter operon, characterized in that the integrative and kanamycin-selectable shuttle vector pMV361 was used.

Also preferred is the protein MptC, characterized in that it was expressed in *M*. *smegmatis.*

The use of the proteins MptAB and/or MptC and/or MptD and/or MptE and/or MptF for the isolation of peptides that specifically bind to said proteins is preferred.

Preferred is an antibody against the M. *paratuberculosis* specific ABC-transporter encoded by the sequence according to SEQ ID NO 1.

Also preferred are antibodies against the proteins MptAB and/or MptC and/or MptD and/or MptE and/or MptF.

Furthermore preferred is the use of the proteins MptAB and/or MptC and/or MptD and/or MptE and/or MptF for manufacturing an antiserum.

Also preferred is a method for the preparation of antisera against at least one of the proteins MptAB or MptC or MptD or MptE or MptF, characterized in that it is raised in an animal. It is furthermore preferred to raise the antibody in a rabbit. In another preferred embodiment, these antisera are obtained by an initial injection of protein MptC, that is followed by two booster injections of recombinant fusion protein, emulsified in adjuvant.

A preferred embodiment of the invention is a therapeutical composition against paratuberculosis and/or Crohn's disease, that contains protein MptAB or protein MptC or protein MptD or protein MptE or protein MptF or at least one fragment of at least one of said proteins or a mixture thereof. In a particularly preferred embodiment, this therapeutical composition is a vaccine, which preferably further contains pharmaceutically acceptable agents, carriers, solvents and/or adjuvants.

According to the invention, a method for inducing an immunological response in an animal or a human is preferred, in which the *M*. *paratuberculosis* specific ABC-transporter operon or at least one of the proteins MptAB or MptC or MptD or MptE or MptF or at least one fragment of at least one of said proteins or a mixture thereof are used to produce antibodies to protect said animal or human from paratuberculosis and/or Crohn's disease.

It was surprisingly found that unique membrane proteins and transport functions are involved in the host preference, the high tenacity, and the iron uptake mechanisms of *M*. *paratuberculosis.* Since a direct approach targeting the identification of *M*. *paratuberculosis*-specific membrane proteins by comparative 2-D-electorphoresis was unlikely to succeed due to the high mechanic stability of mycobacterial membranes, a reverse genetics approach was chosen. A representational difference analysis (RDA; Lisitsyn, N., N. Lisitsyn, and M. Wigler. 1993. Cloning the differences between two complex genomes. Science 259:946-951) was performed followed by sequencing and specific analyses for the presence of open reading frames containing putative membrane-spanning regions. Using this approach we identified and characterized the first *M*. *paratuberculosis* specific membrane protein and expressed it in *M*. *smegmatis* mc²155.

According to the present invention, we cloned and characterized the first *M*. *paratuberculosis* specific membrane protein designated as MptC. The data indicates that this protein is part of an *M*. *paratuberculosis* specific ABC-transporter. The cloned sequence of this transporter encodes at least three proteins, namely MptAB, MptC, and MptD. Both MptAB and MptC contain an amino-terminal transmembrane domain and a carboxy-terminal ATP-binding domain. A similar secondary structure has been described for the putative ABC-transporter proteins YbtP and YbtQ of *Y*. *pestis* (Fetherston, J. D., V. J. Bertolino, and R. D. Perry. 1999) and could also be demonstrated for the putative ATP-binding proteins Rv1348 and Rv1349 of *M*. *tuberculosis* (Braibant, M., P. Gilot, and J. Content. 2000). These two putative ABC-transporter protein encoding genes are followed by a gene encoding the transmembrane protein MptD. Although the MptD protein has no sequence homologies to known proteins, its secondary structure and position correspond to the YbtX protein encoded within the ybt operon immediately downstream from the ybtP and ybtQ genes (Fetherston, J. D., V. J. Bertolino, and R. D. Perry. 1999).

The start codon of the putative MptE protein does not overlap the stop codon of the MptD protein, but is located 78 base pairs (bp) downstream. The protein has only a limited degree of homology to an ABC transporter permease protein of *Lactococus lactis*. The following MptF protein has a significant homology to a structurally different putative ATP-binding protein. As similar arrangements are unknown for other ABC transporters, a functional association of the putative MptE and MptF proteins with the proteins mptAB, mptC, and mptD remains unclear.

The function of the mpt-operon cannot be predicted based on sequence homologies. One of the highly homologous transporters is predicted to have an export function (Rv1348/1349) whereas the other one is known to be required for iron uptake (ybtPQX). Therefore, in order to obtain an initial clue on possible functions, the putative ABC transporter genes were transformed into M. smegmatis mc²155, and a strongly reduced lag phase in the presence of kanamycin was observed. The initial idea that the MptC protein might be involved in a process of efflux-mediated drug resistance, as it has been described for a *M*. *smegmatis* transport system (Banerjee, S. K. , K. Bhatt, P. Misra, and P. K. Chakraborti. 2000. Involvement of a natural transport system in the process of efflux-mediated drug resistance in Mycobacterium smegmatis. Mol.Gen.Genet. 262:949-956) is unlikely, since *M*. *paratuberculosis* strain 6783 has been shown to be kanamycin sensitive. A second hypothesis predicting an involvement in the excretion of regulatory molecules as it has been shown for gram positive and gram negative bacteria (Young, J. and I. B. Holland. 1999. ABC transporters: bacterial exporters-revisited five years on. Biochim.Biophys.Acta 1461:177-200) could also not be confirmed. Thus, preliminary experiments showed no influence of supernatants from M. smegmatis pRDIII320 transformants on the lag phase of pMV361 transformants (data not shown). Another possibility for the function of the Mpt-operon could be an involvement in iron uptake, as suggested for the ybtPQX operon (Fetherston, J. D., V. J. Bertolino, and R. D. Perry. 1999). Such a function has previously been reported for a mycobacterial ABC-transporter involved in exochelin uptake in *M*. *smegmatis* (Zhu, W., J. E. Arceneaux, M. L. Beggs, B. R. Byers, K. D. Eisenach, and M. D. Lundrigan. 1998. Exochelin genes in Mycobacterium smegmatis: identification of an ABC transporter and two non-ribosomal peptide synthetase genes. Mol.Microbiol. 29:629-639). However, for transporters mediating solute uptake the existence of an additional substrate-binding protein located outside the cytoplasmatic membrane is usually required (Fath, M. J. and R. Kolter. 1993. ABC Transporters: Bacterial Exporters. Microbiol. Rev. 57:995-1017), and such a protein is not encoded for on the DNA analysed so far. Since the DNA fragment analyzed, even at the ends, has no significant homology to the *M*. *avium* genome, it might be part of a *M*. *paratuberculosis* specific pathogenicity island, possibly involved in iron uptake, as recently reported for *Streptococcus (S.) pneumoniae* (Brown, J. S., S. M. Gilliland, and D. W. Holden. 2001. A Streptococcus pneumoniae pathogenicity island encoding an ABC transporter involved in iron uptake and virulence. Mol.Microbiol. 40:572-585). This would be particularly interesting as it has been shown that the components of this system can protect mice against systemic *S*. *pneumoniae* infection (Brown, J. S., A. D. Ogunniyi, M. C. Woodrow, D. W. Holden, and J. C. Paton. 2001. Immunization with Components of Two Iron Uptake ABC Transporters Protects Mice against Systemic Streptococcus pneumoniae Infection. Infect.Immun. 69:6702-6706).

According to the invention we have cloned and characterized the first *M*. *paratuberculosis* specific membrane protein MptC. By showing that it most likely is part of an ABC-transporter operon which again might be part of a pathogenicity island, we have made a first step towards investigating the cause for the distinct features of *M*. *paratuberculosis* on a molecular basis.

### Examples

The following examples describe the present invention further.

### The following materials and methods were used:

### Materials

Bacterial strains, plasmids, primers and growth conditions. The bacterial strains used in this study are listed in Table 1, plasmids and primers are noted in Table 2. Mycobacteria were grown on Middlebrook 7H10 agar (MB-agar) or in Middlebrook 7H9 medium (MB-medium; both DIFCO Laboratories, Detroit, MI) supplemented with OADC enrichment (DIFCO) , Tween 80® (0.05 %) and the appropriate antibiotics (kanamycin, 40 µg ml-1) for *M*. *smegmatis* transformants; for *M*. *paratuberculosis* mycobactin (2 µg ml⁻¹; Synbiotics, Lyon, France) was added. *Escherichia* (*E*.) *coli* strains were grown in Luria Bertani (LB) medium supplemented with the appropriate antibiotics (ampicillin, 100 µg ml⁻¹; kanamycin, 40 µg ml⁻¹) .

### Representational difference analysis (RDA) and isolation of RDA fragments

Mycobacterial chromosomal DNA was extracted according to the method described by Bose et al. (Bose, M., A. Chander, and R. H. Das. 1993. A rapid and gentle method for the isolation of genomic DNA from mycobacteria. Nucleic Acids Res. 21:2529-2530 ). Then the RDA-technique (Lisitsyn, N., N. Lisitsyn, and M. Wigler. 1993) recently applied to isolate a *M*. *paratuberculosis*-specific low G+C content island (Tizard, M., T. Bull, D. Millar, T. Doran, H. Martin, N. Sumar, J. Ford, and J. Hermon-Taylor. 1998. A low G+C content genetic island in Mycobacterium avium subsp. paratuberculosis and M. avium subsp. silvaticum with homologous genes in *Mycobacterium tuberculosis*. Microbiology 144:3413-3423.) and the novel *M*. *paratubercul*sosis-specific insertion sequence like element ISMav2 (Strommenger, B., K. Stevenson, and G. F. Gerlach. 2001), was used as previously described (Strommenger, B., K. Stevenson, and G. F. Gerlach. 2001).

### Manipulation and analysis of DNA

Gel electrophoresis, Southern blot, plasmid preparation, PCR, DNA cloning, and transformation of *E*. *coli* were done following standard procedures (Sambrook, J., E. F. Fritsch, and T. Maniatis. 1989. Molecular cloning: a laboratory manual, 2nd ed. Cold Sping Harbor Laboratory Press, Cold Spring Harbor Laboratory, N.Y.). DNA modifying enzymes were puchased from New England Biolabs (Karlsruhe, Germany). Nucleotide sequencing was done by "primer walking"; primers were purchased from MWG Biotech AG (Ebersberg, Germany), and sequencing reactions were done by SeqLab (Göttingen, Germany). Sequencing data analyses were performed using the HUSAR 5.0 programme (dkfz, Heidelberg, Germany). The DNA sequence as well as the putative open reading frames were investigated for homologies in GenBank / EMBL- as well as in the TIGR and Sanger Center data bases.

### Construction of recombinant mycobacterial shuttle plasmids and deletion derivatives and their expression in M. smegmatis mc²155

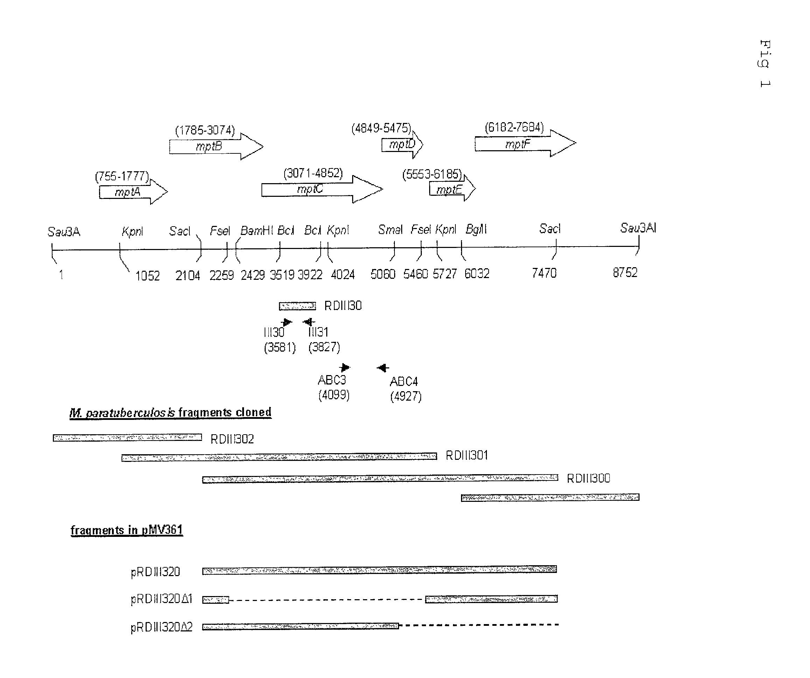

The 5367 bp SacI-fragment RDIII300 (Fig. 1), comprising the complete ORFs for mptC, mptD and mptE and the incomplete ORFs for mptB and mptF, was cloned into the mycobacterial shuttle vector pMV361 (Tab. 2) under control of the vector-based hsp60 promoter. Based on this plasmid, designated pRDIII320, deletion derivatives were generated. Deletion of the 3201 bp FseI fragment (position no. 1208 to 4409 in Fig. 1), comprising the ORFs mptB, mptC and mptD resulted in plasmid pRDIII320D1. Deletion of the DNA downstream of the SmaI restriction site at position no. 4009 (Fig. 1) resulted in plasmid pRDIII320D2 lacking the ORFs mptE, mptF, and part of ORF mptD (Table 2). These plasmids were used to transform *M*. *smegmatis* mc²155 by electroporation as described by Sander and Boettger (Sander, P. and E. C. Boettger. 1998. Gene Replacement in Mycobacterium smegmatis Using a Dominant Negative Selectable Marker, p. 207-216. In T. Parish and N. G. Stoker (eds.), Mycobacteria Protocols. Humana Press Inc., Totowa, New Jersey, USA.). Briefly, 250 ml cultures of *M*. *smegmatis* mc²155 were incubated in a roller bottle at 37 °C until an optical density at 660 nm (OD₆₆₀) of 0.4 was reached. Cultures were placed on ice for 90 min, and bacteria were harvested by centrifugation (2.600 x g) for 15 min at 4 °C. Pellets were washed twice with glycerol (10%) and finally resuspended in 2.5 ml of glycerol (10%). For transformation, 100 µl of cells were mixed with 0.5 µg DNA and incubated on ice for 10 min before transferring into an electroporation cuvette. Electroporation was performed with a single pulse using the following settings: 2.5 kV, 1000 W and 25 µF. Bacterial cells were resuspended in MB-media immediately after electroporation and incubated on a rotating shaker at 37 °C for 2 hours before plating on agar plates containing kanamycin (40 µg ml⁻¹) for positive selection. The success of the transformations was controlled by PCR with primers specific to mptC (IPIII30 and IPIII31, Table 2, Fig. 1) and for the kanamycin resistence determinant (Kan3 and Kan4, Table 2), as well as by Southern blot analyses.

### Preparation of antiserum

A DNA fragment obtained by PCR using the primers ABC3 and ABC4 was cut with BamHI and EcoRI and cloned into pGEX5x-3 resulting in plasmid pRDIII350 (Table 2). Upon induction with isopropyl-thiogalactoside (IPTG; 1 mM final concentration) inclusion bodies were formed and purified as described previously (Gerlach, G. F., C. Anderson, A. A. Potter, S. Klashinsky, and P. J. Willson. 1992. Cloning and expression of a transferrin-binding protein from Actinobacillus pleuropneumoniae. Infect.Immun. 60:892-898). Serum against the MptC protein was raised in rabbits by an initial intra-cutaneous injection and two booster injections using 100 µg of recombinant fusion protein emulsified in adjuvant (Emulsigen-Plus®, MVP Inc., Ralston, Nebr.).

### Protein preparations, electrophoresis and Western Blotting

Mycobacterial whole cell lysates were prepared from 100 mg of bacterial pellet. Bacteria were resuspended in 500 µl H₂O, and cell disruption was achieved by mechanical treatment for 190 sec with zirconium beads in a mini-bead beater (Bio-Spec Products, Inc., Bartlesville, Okla.) followed by sonication. Cell debris was removed by centrifugation (2 500 x g) for 5 min. Mycobacterial membranes were prepared according to the protocol of Hancock and Nikaido (Hancock, R. E. and H. Nikaido. 1978. Outer membranes of gram-negative bacteria. XIX. Isolation from Pseudomonas aeruginosa PAO1 and use in reconstitution and definition of the permeability barrier. J.Bacteriol. 136:381-390). Briefly, 100 mg of bacterial pellet was resuspended in 2 ml Tris-HCl (30 mM, pH 8.0) containing 20% saccharose. Cell disruption was achieved as described above. Residual cellular debris was removed by centrifugation (17 000 x g) for 10 min; total mycobacterial membranes were obtained by ultracentrifugation (175 000 x g) for 2 h at 4 °C and dissolved in 500 µl TE buffer. Protein aggregates were prepared as peviously described (Gerlach, G. F., C. Anderson, A. A. Potter, S. Klashinsky, and P. J. Willson. 1992). All protein preparations were analyzed by sodium dodecyl sulfate-polyacrylamide gel electrophoresis (SDS-PAGE) and Western Blot analysis as described earlier (Homuth, M., P. Valentin-Weigand, M. Rohde, and G. F. Gerlach. 1998. Identification and characterization of a novel extracellular ferric reductase from Mycobacterium paratuberculosis. Infect.Immun. 66:710-716.). For the detection of the MptC protein in *M*. *paratuberculosis* the ECL Western blotting detection system® (Amersham Pharmacia Biotech, Freiburg) was used according to the manufacturer's instructions; for other Western blots an alkaline-phosphatase-labelled goat anti-rabbit conjugate and a substrate containing Nitro Blue Tetrazolium and 5-bromo-4-chloro-3-indolyl phosphate was used (Gerlach, G. F., C. Anderson, A. A. Potter, S. Klashinsky, and P. J. Willson. 1992).

### Determination of growth kinetics

Material from cultures of *M*. *smegmatis* mc²155 transformants grown on MB-agar containing kanamycin was resuspended in MB-medium and adjusted to an optical densitiy (OD₆₆₀) of 0.5. Liquid cultures (2.5 ml) with and without kanamycin were inoculated with 5 µl of this suspension and incubated on a rolling incubator at 37 °C. The OD₆₆₀ was determined at various time points.

The present invention is further illustrated by the figures 1 to 5, together with the detailed description of the invention.

Figure 1 shows the physical map of the mptC gene and adjacent sequences. The open arrows above indicate the size of the ORFs mptA-F; the numbers in parentheses indicate the position of start and stop codon. The grey bars underneath indicate the size and relative location of the DNA fragments originally cloned. The open bars indicate the fragments cloned into the vector pMV361 and transformed into M. *smegmatis* mc²155. The small arrows indicate the primers used in the examples of the present invention study; the position of the 5'-end is given in parentheses.

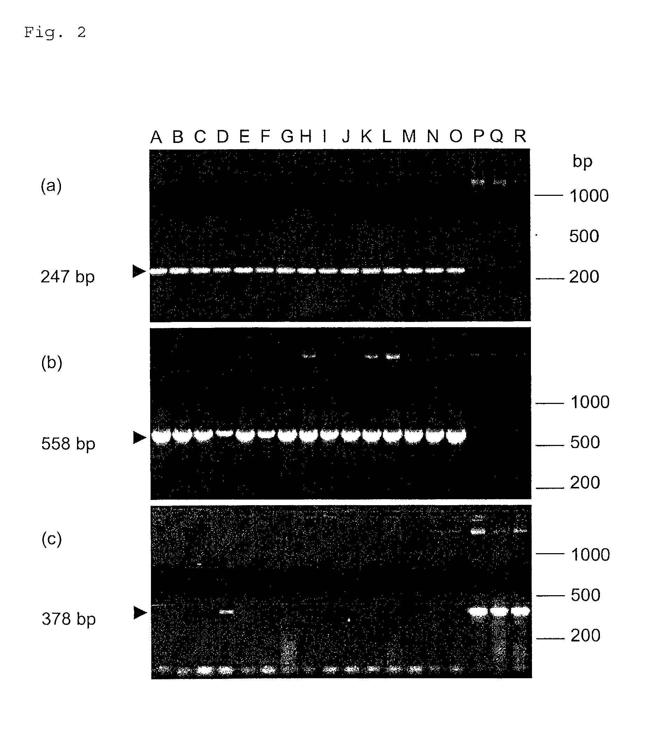

Figure 2 shows PCR-analyses of *M*. *paratuberculosis* and *M*. *avium* isolates using (a) primers IPIII30 and IPIII31, (b) primers MK05 and MK06, (c) primers MK07 and MK08. The template DNA used is derived from 14 bovine clinical M. paratuberculosis isolates (lanes A to N), M. paratuberculosis strain 6783 (tester; lane O), M. *avium* strain ATCC 25291 (driver; lane P), M. avium strain DSM 44157 (lane Q), and M. *avium* strain DSM 44158 (lane R).

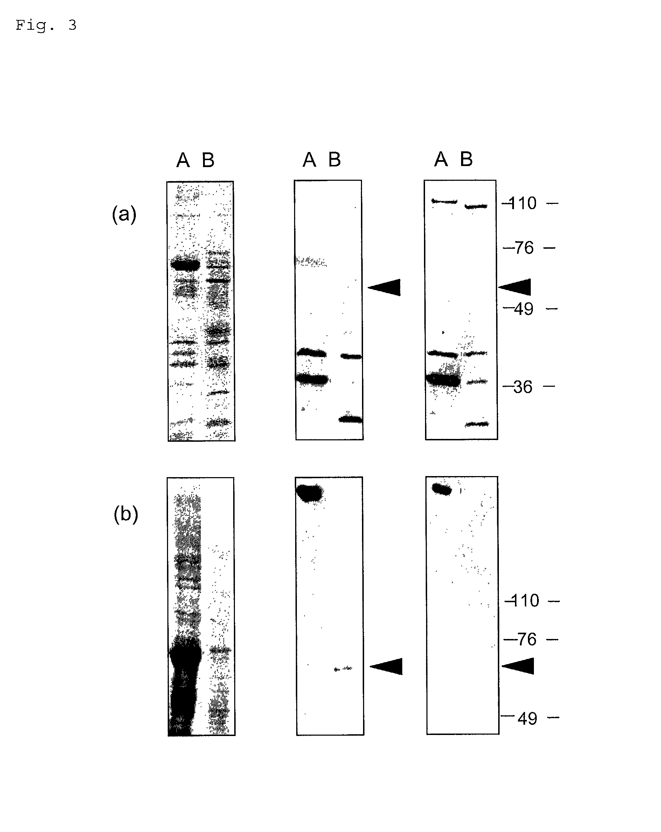

In Figure 3, Coomassie blue-stained gels (left) and Western blots using serum raised against the recombinant MptC fusion protein and developed with (a) alkaline-phosphatase-labelled goat anti-rabbit conjugate and a substrate containing Nitro Blue Tetrazolium and 5-bromo-4-chloro-3-indolyl phosphate or (b) the ECL-system are shown. The serum used is absorbed with whole cell lysates of Escherichia coli transformants containing pGEX5x-3 (left hand blot) or pRDIII350 (right hand blot). In lane A, membranes prepared from *M*. *smegmatis* mc²155 were loaded, in lane B, membranes from *M*. *paratuberculosis* strain 6783 were loaded. The numbers on the right indicate the relative positions of size markers in kilodaltons. The arrowheads indicate the expected position of the MptC protein (64 kilodaltons (kDa)).

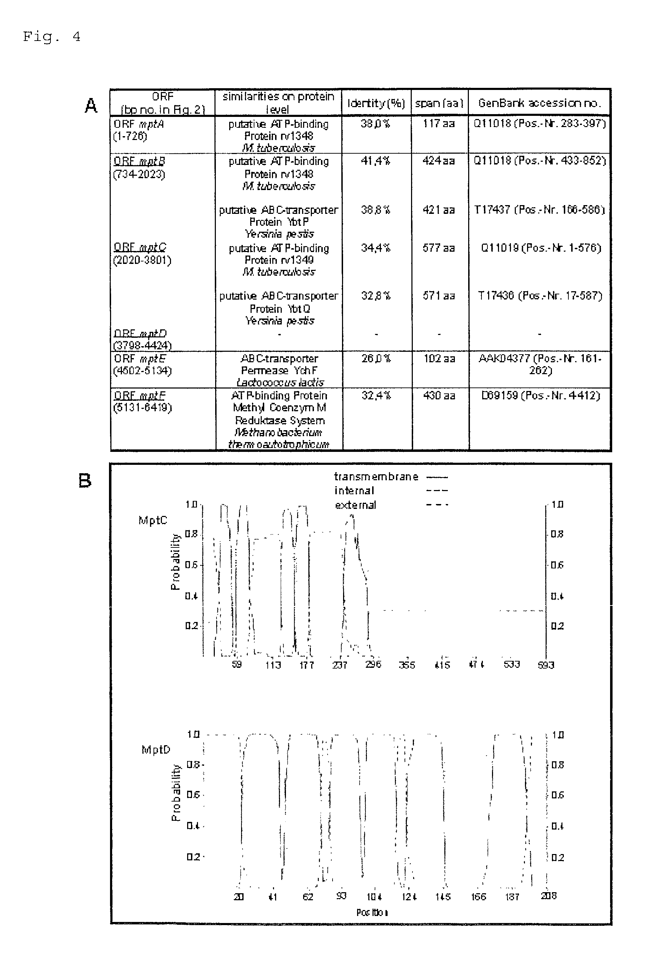

Figure 4 depicts the analysis of the Mpt proteins. (A) Sequence identities as determined by BLAST search and (B) prediction of transmembrane helices in the proteins MptC and MptD (Sonnhammer, E. L., G. von Heijne, and A. Krogh. 1998) .

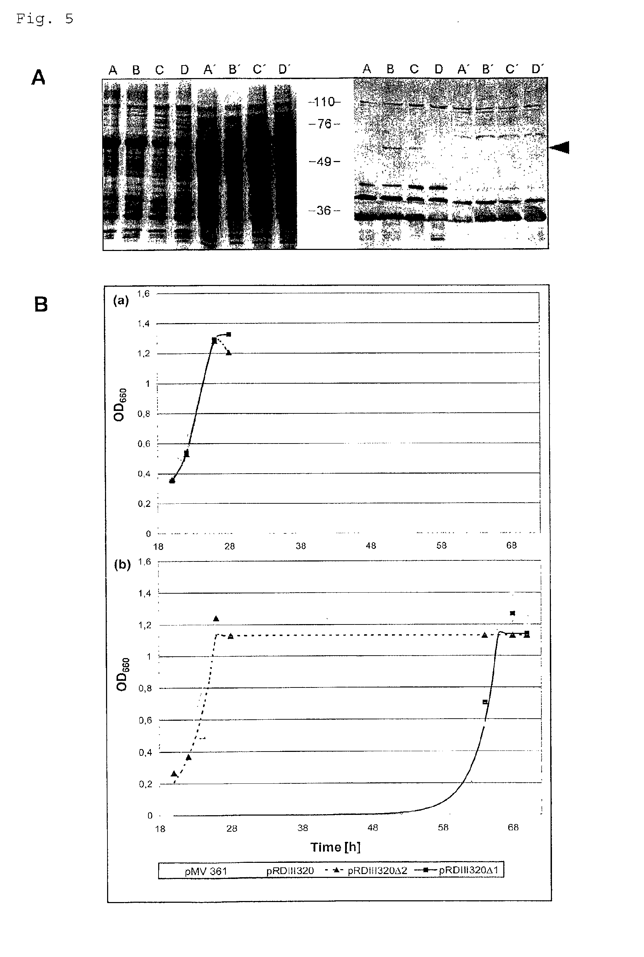

Figure 5 shows analysis of *M*. *smegmatis* mc²155 transformants. (A) Coomassie blue-stained gel (left) and Western blot (right) of membrane preparations (A-D) and whole cell lysates (A' - D') using serum raised against the recombinant MptC fusion protein. Proteins are prepared from cells transformed with pMV361 (A), pRDIII320 (B), pRDIII320Δ2 (C), pRDIII320Δ1 (D). The numbers in the middle indicate the relative positions of size markers in kilodaltons. The arrowhead to the right indicates the expected position of the MptC protein (64 kDa). (B) Growth curves of *M*. *smegmatis* mc²155 transformants in MB medium without (top) and with the addition of kanamycin (40 µg ml⁻¹; bottom).

### RDA-based isolation of the M. paratuberculosis specific DNA fragment RDIII30

Among several fragments cloned by an RDA approach, a 403 bp BclI-fragment designated as RDIII30 was identified to encode putative transmembrane domains by DNA sequencing and analysis of the predicted amino acid sequence with the programme TMHMM (Sonnhammer, E. L., G. von Heijne, and A. Krogh. 1998. A hidden Markov model for predicting transmembrane helices in protein sequences. Proc. Int. Conf. Intell. Syst. Mol. Biol. 6:175-182). Data base comparison did not show a significant degree of DNA sequence homology to other mycobacterial species. Subspecies specificity of the sequence was further investigated by PCR analyses using the internal primers IPIII30 and IPIII31 (Table 2). A single amplification product of the predicted size was obtained for all seventy-nine *M*. *paratuberculosis* strains tested; for the nine IS901-positive *M*. *avium* strains and for the reference strains of nine other mycobacterial species tested no amplification product was obtained (Fig. 2). These results supported the M. *paratuberculosis* specificity of fragment RDIII30.

### Isolation of large M. paratuberculosis specific DNA fragments

Using RDIII30 as a probe, two overlapping fragments were cloned from *M*. *paratuberculosis* strain 6783. One of these fragments was a 5367 bp SacI fragment designated as RDIII300, the other a 4681 bp KpnI fragment designated as RDIII301 (Fig. 1). The two fragments together span 6419 bp of DNA; sequence comparison with the known genome sequence of *M*. *avium* in a BLAST search using the TIGR database (www.tigr.org) showed an overall identity of the segment (including both ends) with the *M*. *avium* genome of only 60%. Analysis of the DNA sequence revealed the presence of six ORFs, designated mptA to mptF, with mptA and mptF being present only partially on the DNA fragment analyzed. The ORFs mptB, mptC and mptD as well as mptE and mptF directly follow each other with start and stop codons overlapping. The original RDA fragment RDIII30 is part of mptC coding for a predicted protein (MptC) of 593 amino acids in length and a calculated molecular mass of 64 kDa (Fig. 1).

### Expression of the MptC protein in M. paratuberculosis

In order to investigate the expression of mptC by *M*. *paratuberculosis,* a serum raised against a GST-MptC fusion protein was used to determine expression of MptC in *M*. *paratuberculosis* and in *M*. *smegmatis* (negative control). Using a highly sensitive chemiluminescence detection system, the expression of a 64 kDa protein was detected in *M*. *paratuberculosis* membrane preparations only (Fig. 3), demonstrating that the MptC protein is expressed by *M*. *paratuberculosis* grown in standard culture conditions. The absence of a specific serological reaction with M. smegmatis membranes further supports the subspecies specificity of the Mpt protein.

### Sequence analysis

A data base search at the protein level (Fig. 4A) showed that the MptC protein had obvious similarities to the putative ATP-binding proteins Rv1349 from *M*. *tuberculosis* (34.4%; GenBank accession no. Q11019 (Braibant, M., P. Gilot, and J. Content. 2000. The ATP binding cassette (ABC) transport systems of Mycobacterium tuberculosis. FEMS Microbiol.Rev. 24:449-467) and YbtQ from Yersinia (*Y*.) pestis (38.2% GenBank accession no. T17436, (Fetherston, J. D., V. J. Bertolino, and R. D. Perry. 1999. YbtP and YbtQ: two ABC transporters required for iron uptake in Yersinia pestis. Mol.Microbiol. 32:289-299). The protein encoded by the ORF mptB, located upstream from mptC (Fig. 2), showed 41.4% similarity to another putative ATP-binding protein (Rv1348) of *M*. *tuberculosis* (GenBank accession no. Q11018; Braibant, M., P. Gilot, and J. Content. 2000) and 38.8% similarity to another ABC-transporter protein YbtP from Y. pestis (GenBank accession no. T17437; Fetherston, J. D., V. J. Bertolino, and R. D. Perry. 1999) ; both genes are located upstream of the respective mptC homologues. The similarity between the protein encoded by ORF mptB and Rv1348 is limited to the carboxy-terminal part of the putative ATP-binding protein Rv1348, whereas the protein encoded by ORF mptA shows 38% identity over 117 amino acids to the amino-terminal part of Rv1348. Based on these observations it can be hypothesized that the ORFs mptA and mptB encode a single protein MptAB via a programmed translational frameshift event occuring at a "slippery site" (CCC CCA; Snyder, L. and W. Champness. 1997. Exceptions to the code, p. 55. In Molecular Genetics of Bacteria. ASM Press, Washington, D.C.) present 30 bp upstream of the start codon of ORF mptB. A similar translational frameshift event resulting in the expression of a single transporter protein has recently described by Barsom and Hatfull (Barsom, E. K. and G. F. Hatfull. 1997. A putative ABC-transport operon of Mycobacterium smegmatis. Gene 185:127-132) for a permease protein of an ABC-transporter operon of *M*. *smegmatis.*

While the protein encoded by ORF mptF shows 32.4% similarity over 430 amino acids to an ATP-binding protein of *Methanobacterium thermoautotrophicum,* the proteins encoded by the ORFs mptD and mptE exhibit only weak or no similarities to known proteins (Fig. 4A). Analyses of the amino acid sequences of the putative Mpt proteins for the presence of conserved sequence motifs of ABC-transporter proteins revealed that the putative proteins MptAB and MptC comprised all typical sequence motifs, i.e. the Walker A- and Walker B-motif, the ABC-signature and the Linton and Higgins-motif (Linton, K. J. and C. F. Higgins. 1998. The Escherichia coli ATP-binding cassette (ABC) proteins. Mol.Microbiol. 28:5-13), whereas in the incomplete putative protein MptF only the Walker A-motif and the ABC-signature were found. However, the lacking motifs might be located on the unknown part of the ORF. Using the programme TMHMM (Sonnhammer, E. L., G. von Heijne, and A. Krogh. 1998.) to identify putative transmembrane domains, membrane spanning domains consisting of 5 (MptAB) or 6 to 7 (MptC) transmembrane helices were predicted for the proteins MptAB (data not shown) and MptC (Fig. 4B). The putative protein MptD was predicted to be a transmembrane protein comprising six transmembrane helices (Fig. 4B). Based on these findings the 6419 bp DNA fragment described was considered to be part of a putative ABC-transporter system. The cloned part of the system was assigned the GenBank accession no. AF419325. It encompasses two putative ABC-transporter proteins, each of them consisting of a carboxyterminal ATP-binding domain fused to an N-terminal transmembrane domain (MptAB and MptC), followed by a transmembrane protein (MptD) and two additional putative proteins (MptE and MptF) with uncertain function.

### Expression of the MptC protein in M. smegmatis

The MptC protein was not expressed in E. coli transformants. In order to confirm the sequence and to investigate a possible function of the MptC protein, fragment RDIII300 (Fig. 1) was cloned into the mycobacterial shuttle vector pMV361, yielding plasmid pRDIII320. *M*. *smegmatis* mc²155 was transformed with this construct as well as with two deletion derivatives (pRDIII320D1 and pRDIII320D2; Table 2; Fig. 1). Using the serum raised against the recombinant GST-MptC fusion protein, the expression of MptC was investigated in whole cell lysates and membrane preparations of the *M*. *smegmatis* transformants. In membrane preparations of pRDIII320 and pRDI-II320D2 transformants (both containing ORF mptC) the expression of a 64 kDa protein was demonstrated (Fig. 5A). In order to investigate possible influences of MptC expression on the phenotype of *M*. *smegmatis* transformants, growth kinetics were determined in medium with and without kanamycin (Fig. 5B). In medium without kanamycin cultures of all *M*. *smegmatis* transformants showed similar growth kinetics with visible growth after 20 to 24 hours, reaching the maximum OD₆₆₀ 4 to 6 hours later. In medium containing kanamycin only, *M*. *smegmatis* transformants carrying the plasmids pRDIII320 and pRDIII320D2 showed these growth kinetics; cultures of the pMV361 and pRDIII320D1 transformants had a clearly prolonged lagphase with visible growth not occurring until 60 hours after inoculation and then also reaching the maximum OD₆₆₀ within 4 to 6 hours.

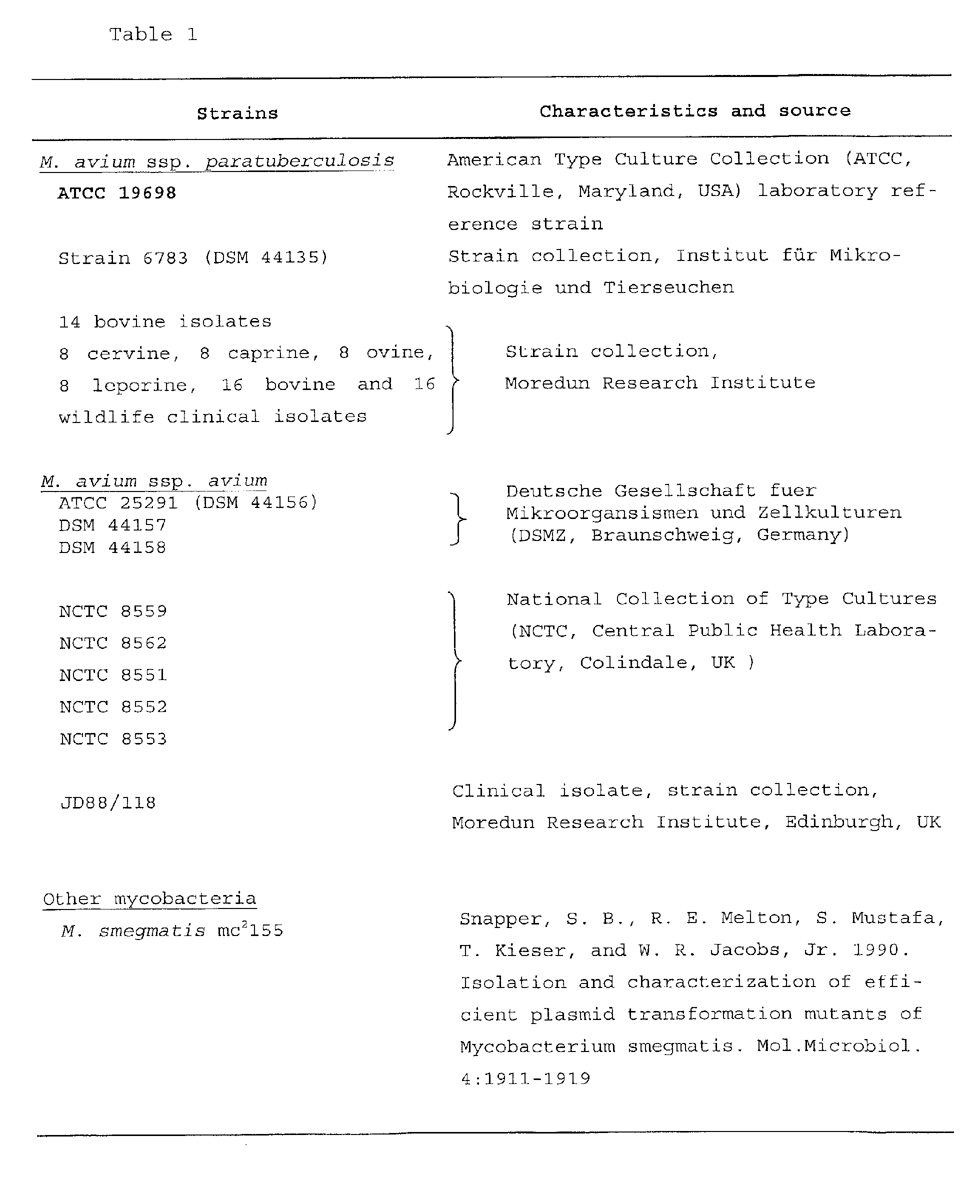

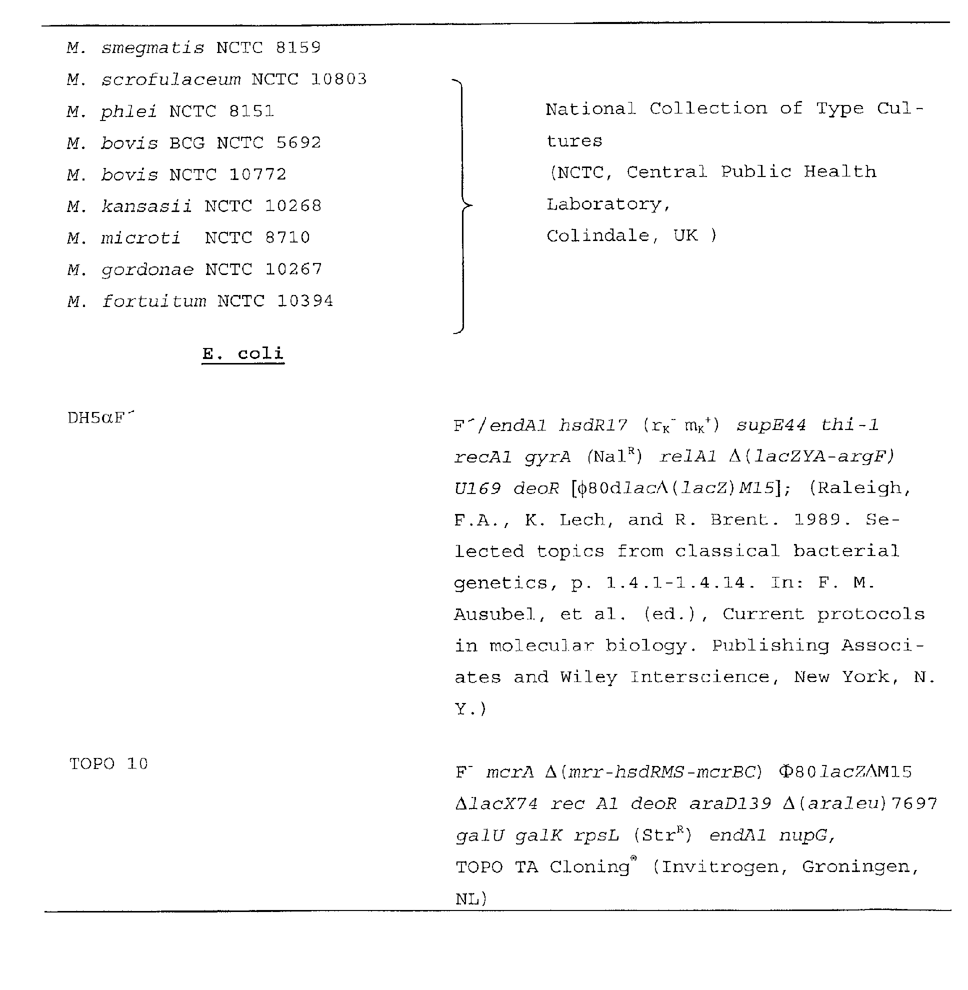

**Table 2**

| Plasmids and primers | Characteristics, reference or source |
|---|---|
| Plasmids | |
| pUC19 | *E*. *coli* cloning vector carrying an ampicillin resistance determinant, Pharmacia |
| pCR® II-TOPO | *E*. *coli* cloning vector carrying an ampicillin and kanamycin resistance determinant, Invitrogen |
| pGEX5x-3 | *E*. *coli* expression vector for the construction of glutathion-S-transferase (GST) fusion proteins, Pharmacia Integrative mycobacterial shuttlevector carrying a kanamycin resistance determinant (Burlein, J. E., C.K. Stover, S. Offutt, and M.S. Hanson. 1994. Exression of Foreign Genes in |
| pMV361 | Mycobacteria, p. 239-252. In B. R. Bloom (ed.), Tuberculosis: Pathogenesis, Protection and Control. ASM Press, Washington,D.C.) |
| pRDIII300 | fragment RDIII300 in pUC19 |
| pRDIII301 | fragment RDIII301 in pUC19 |
| pRDIII320 | fragment RDIII300 in pMV361 |
| pRDIII320Δ1 | deletion derivative of pRDIII320 with a 3201 bp *Fse*I fragment deleted (Fig. 1) |
| pRDIII320Δ2 | deletion derivative of pRDIII320 with a 2410 bp *Sma*I/*Sac*I fragment deleted (Fig. 1) |
| pRDIII350 | PCR-fragment obtained with primers ABC3 and ABC4, cut with BamHI and EcoRI and ligated into pGEX5x-3. |

| Primers | |
|---|---|
| RBam12 | GAT CCT CGG TGA (Lisitsyn, N., N. Lisitsyn, and M. Wigler. 1993. Cloning the differences between two complex genomes. Science 259:946-951) |
| RBam24 | AGC ACT CTC CAG CCT CTC ACC GAG (Lisitsyn, N., N. Lisitsyn, and M. Wigler. 1993. Cloning the differences between two complex genomes. Science 259:946-951) TTC TTG AAG GGT GTT CGG GGC C (Doran, T.J., J.K. Davies, |
| MK5 | A.J. Radford, and A. L. Hodgson. 1994. Putative functional domain within ORF2 on the Mycobacterium insertion sequences IS900 and IS902. Immunol. Cell Biol. 72:427-434) GCG ATG ATC GCA GCG TCT TTG G (Doran, T.J., J.K. Davies, |
| MN6 | A.J. Radford, and A. L. Hodgson. 1994. Putative functional domain within ORF2 on the Mycobacterium insertion sequences IS900 and IS902. Immunol.Cell Biol. 72:427-434) GTC TGG GAT TGG ATG TCC TG (Kunze, Z.M., S. Wall, R. Appel-berg, M.T. Silva, F. Portaels, and J.J. McFadden. 1991. |
| MK7 | IS901, a new member of a widespread class of atypical insertion sequences, is associated with pathogenicity in Mycobacterium avium. Mol. Microbiol. 5:2265-2272) CAC CAC GTG GTT AGC AAT CC (Kunze, Z. M., S. Wall, R. Appel-berg, M. T. Silva, F. Portaels, and J. J. McFadden. 1991. |
| MK8 | IS901, a new member of a widespread class of atypical insertion sequences, is associated with pathogenicity in Mycobacterium avium. Mol.Microbiol. 5:2265-2272) |
| ABC3 | CCG CGG ATC CGC TTA CGA CGG AGG TCA A |
| ABC4 | 5GCC GGA ATT CGA TGT TGA TGA GAAT CCC T |
| IPIII30 | CTA TGC GCA CTG ACG CTT C |
| IPIII31 | TTC CGA AGA ATC CGA TGA G |
| Kan3 | CTC ATC GAG CAT CAA ATG AAA CTG C |
| Kan4 | ATA TTC AAC GGG AAA CGT CTT GCT C |

## Claims

1. *M*. *paratuberculosis* specific ABC-transporter operon with the sequenze (SEQ ID NO 1)

GATCCATGAGGTTCATGACGAAGTTCTCCCAGGCGCTCAACAGGTCGCCGAA
AAAACCTCCGCCGCCGGAAGAGTCGCCGGTGTCTCCCGCCGATGAGTCCTCG
CTTTTCACAATTGCCGGCGCTGCGCTGCTGCGTGGCGCCGCCGCCAATGTGC
TGGTGGAGACGGTCTGATACGTACTCATCGTGGTGGCGGCCTGGACCCACAT
TCGTGCGTAGTCGGCCTCGTTGAGCGCGATCGGGATCGTGTTGATCCCGAAG
AAGTTGGTTGCCACCAGCGCCGCGTGAGTAGCGTGGTTCGTCGCTAGCTCGG
GCAAGGTGGGCATGGTGGCCAGTGCGACTGTGTAGGCGCTGGCCGCAATCTC
GTGCTGGGCAGCCTCCCCCGCGGCGTCGGCACTGGACTGGTCCAACCACGCT
AGATAGGGAGCATGTGCGGCCACGTACGCCTCGGTGGTCGGGCCCTCCCACG
GACCGGCTTGGACCGCCGCAGCGTCGCGGTGAGTTCGTCTGCGGCCGAGGCG
TATTCAGCGCTCAGCGACGACCAAGCGGCGGCCGCTTCCAGCAGTGGCCCCG
GCCCGGGTCCGCTGCTCAACAGTGCCGAGTGAATCTCTGGCGGCGAAGCCAT
CCACACCGGGGCTGTCATCGCCCACCACCCTTGGCAGAGGACGACAGGACTG
CTGCATCAGCGGTCGCATCGAGAACCGCCATTGCGGTGGCGAGCGGCGACGC
CATCGCGCGCGGTTGGCGCTGAACTTGTGAGATTCGACGTGATATGGGCACG
GGCTGATCCCCTTTCATCACATGCAAACACCCCCGTAATGAAAATCATTTTC
ATTACGGACGGGGACCCTACGGCGCTGAGACCTTTAATGAAAGTCATTTTCA
ATAACCCAGATGTGACGGAGGTCACACTGGTCGCGGGTGCGCGCCGTGGCG
CTCGGCGTCCGATGCGCGCTTGACCGCTGATCGAATGCCCTGCCCGAGGTTT
GGCCGCGGGGGCTGCGATGCCTTGCGGTCGTGGCAATGCTCGGATTCCACA
GCCTTCACGCCGGTACCCCTTATGCGCGCGCCGCCAGAGCGCCCGGAGGC
GAATTCCGCTGCGCGCCGGAGTTTTCAGCTCTCTGGGCGCAGGCACAGCCAT
CGGATTAATCGGCGTTCGCCGCGCGGGCTCGCCCTACTGAGCGTGTCGGAAG
GGACGGCTCCCTACCCACTGATCCATCCCTTGCGTGGAGGTATGTTATCGAT
AACGATTTTCATTATCGGTTCACGATGGCTCGGTTTGAGCGCGGAAGGATTG
AGCATGGACCCGAATACGCCATCACAAGTTGGGCCGCAATCCCCGGTTTTCG
GCGCTGCCCGCGCCACGCTCATTGGATGCGCGCTGTTGACCGCACTCAGCGC
CCTTGCCGGGATGGCACCGCTGATCGCGGTGGTGGAGGTCAGCCGACGGCTA
CTAGCTGGTGACACTGCTGTTTGGGCGATCATTGCAGCGGCGCTCGTTGCGC
TCGCCGTCAAGCAGCTTGGTACGTTTTGCGCTGGCGTTCTGACCCACCTGGC

TGACATCCAGGTCTCGTTTCGAATTCGACGAGATTTGCTCGTGAAGCTGAGC
AAACTGCCACTCGGCTGGTTTACCGACCGCAACTCCGGGATTGTCAAGAAGA
CCGTTGAAGACGACGTCAGCGGCCCTGCACCAGCTGATCGCCCATTCCATCG
TGGAACTCACCGCGGCGACAGTTCCCCCATTAGTCGCCTTGACCTACCTGTT
TGCTGTTGACTGGCGCATGGCGCTCGTCACTTTGCTCCCGGTACTGATTGGG
CTGGTCATGTATCAGCGCGCGATGGCCAGCGCGGACACCAAGTATCCAGAGT
TCATGCAGTGGCTGGCCCGACTCAACAGCGCTGCGGTGGAGTTCGTGAACGG
GATCGGCGTCGTCAAAGCGTTCGGCACGCCAGGTGTGGCGAGCCGCCGCTTT
CAGGAAGTGTCGCGTGCCTTTGCGCACTTTTTCTCGACTGGGCCAAATCCA
CCAGCGTCGCAGCGGTTATCGCCGAAACGCTACTGTCGCCGCCCAGCATTCT
GGTTGTCGTCGCGGGGGCCGGCGGAGCTCTGACCGCCAACGGAAGCTGCCG
CTGGATAGCTTTATCGCGTTCCTGGTTTTCGGGACTGTGATAACCGCCGGTC
TGATGACCGTGATGATGTCGATACACCCGCTGGTCACTGCCCTGGAGTCGC
ACGCGATATCCGCGAACTGCTGGCCGGCCCGGAGCTGCCGACACCGGATGAG
CCGGTCGGTCTCGATCCATCCAGCGCCGCGCCGGTGGTGCGCCTGCGTGACG
TGGTGTTCACCTACGGTGAGACCGTTGCGCTGTCCGGCATCAACCTCGAACT
CGACCGCAACACCATTACAGCGTTGGTCGGCCCTTCCGGATCCGGGAAATCG
ACCCTCGCCAAGTTACTGCCCCGGTTCGACGATCCGGTCGAAGGTTCGGTTG
AGCTTTACGGTGTGGACCTACGCAGCGTCGACCCCGCCGAGTTGTATCGACA
TGTCGCTTTCGTGTTTCAAGACGCCGAACTGTTACGCATGAGCATCCGCGAC
AACATCCGACTCAgCCGGCCGGACGCCGACGACTCGCGGTCCGCGACGCCGC
GGACAGGGCCCAAATCCTAGAGCGGATCGAGCAATTGCCGCGCGGCTTTGAT
TCGGTTGTGGGCGAGGACGCCGAGCTTTCCGGGGGCGAACGGCAGCGCGTCT
GCATCGCCCGCGCCATCTTGGCCGACCGCCCCGTGCTGGTCCTCGACGAGGC
CACGGCCAGCGCCGATCCTGAAAACGAAGCTCGAATCCAGGACGCGCTCAAC
GAAGCCGCTTCGGGCCGGACGGTATTGGTAATCGCCCATCGCCTCAACACCA
TTCGTGGCGCTGACCACATCGTCGTACTCGACGACGGCCGAATCGCCGAACA
AGGCACCCATTTCGAGCTTTTGGAGAGCAAAGGACTTTATGCGCGTTTGTGG
GAGCATGACCGTCGTGTTCAGAGCGGCGTCGCCGAACACATGGAGGTTGGGC
GATGAGCACGATGATTCGTTATTTGCTTGAATTACTTGACGAACGCGGTCGG
CGTCACCTTCGCGTGATGCTTGCCTTGCAAGCCGCACAGGGAATTCTGCAGG
GACTCGGTTTCCTTTTCGTCGTACCGCTGATGGGTGTGCTCATCCACAAGCC
GGTTGATACGCACGCCCTTTGGTGCTGGATTGTGGCGATCGCCGTCGCTGTC
GTCGCTCACCACGGACTGCTTGCGTGGAGCACCTCGCTGGGTTACCTGGTCG
GCACAGATGTGCTGACCAGCTTTCACACCCGCATCGGCAACCATCTCGCCAC

GCTGCCAATCGGCTGGTTCCGCACCGATCGCACCGGACCGATCGGGCGTCTG
CTCAGCAAGGGGACCATGGACGTCGCGGACCTTCCCGCGCACCTGCTGCGGC
ATGTCATCGTGGGTGTAGCCGCGCCTGCCACCATGATCATCGGAAGCTATGT
GCTGGACTGGCGGGTCGGGCTCGCATTCACCGCCGGCGCGCTGCTATGCGCA
CTGACGCTTCGACTACTCATGGTCGTCATCCACCGCAACGACGACCAGTACG
ATCACGACATCGGAGTGACCGCGTCACGCATCGTCGAGTACGCCCGTCAGCA
GCCGACACTGCGTGCATACGGCGTCCTGAACCGGCCCGGACTGGAACGCTA
GAGGAATCGCTTGTCCGGCAACAGACTTCGCAGTCCCGCTTGACCATTCGCG
GCGCGTGGGCGCTCATCGGATTCTTCGGAACCGTCCAACTGGTGGTGACCGC
CATCATCGCGCTCACCGTCGCTCTCGCAGTAAGCGGCAGCCTTGCGCTTTCG
ACGATGGTCGGTTTGCTGATCATCACCTTGCGCATGATCGACCCGATTTCTC
AGCTCGGCGATCTCGCCGGCCATGTCCAGGTCAACGCCGACGCCATCCGCCG
AGTGCGTGAACTGTTGACGGTACCCCCGTTGCCCGAACCCACCCATCCAGGT
CAAGCCACGGGTGCCGACATCGAATTGCAGGGGGTTCGTTTCGCTTACGACG
GAGGTCAACCCACCATCGATGGTATCGACGCCGTCTTTCCACAAGGTAGCCT
GACTGCCGTCGTCGGCCCGTCTGGCGCGGAAAAAGCACGCTGCTCAAACTA
ATCGGGCGGTTCTTCGACGTCGACGAAGGCAGCATCACCATCGGCGGCTGCG
ACATCCGCCAACTCGGCACCGCCGGCGTCTCACATCTCACGGCGCAAGTTTT
CCAAGACGTGTACCTCTTTGAAGGCACAATTGCCGACAACCTGCGGATGGCC
GATCCGCAAGCCACCGATGATGATCTCCGCCACGTCGCCACGATCAGTCGCC
TCGACGAGGTAGTCGATCGCCTTCCCAATGGGTGGAAACCCGCGTTGGTgA
GGCGGGGTCgACGCTGTCCGGCGGAAAAGCAGCGTGTCTCCATAGCGCGCG
CCCTGCTCAAAGACGCACCGGTAGTACTGCTGGACGAGGCGACCGCGGCGCT
GGATGCCGCCAATGAGACCGCCGTTTCCGACGCTATCCACGAACTAGCGCGT
GACCGCACAGTCATCGTTGTCGCACACCGCCTTTCGACCGTGGCGGCCGCCG
ATCAGATACTGGTGCTCGACGGCGGCCGGATCACCGAGCGTGGCCGCCACGA
CACACTGGTGGGTGCGGCGGCACCTACGCCCGCTTCTGGGAAGAACGAACT
CGCGCGCGAGGGTGGCAACTGCGCCAAATTGCGCCCACTCCTGAACTGCAGG
CACCTCGACCATGACGGCCACTAGCTCGACGACCCAGTCCAGTCGCCGCATC
GACGTCCGGATGAGCGCCAGGGATCTCATCAACATCGGGGTCTTCGGCGCTC
TCTACATCGCCACTGTGTTCGCGATCAACGTGTTCGCTTTCATCAATCCGCT
CGTCATGTTGGTCGCCCTGGCGGTCAGCATGATCGCCGGCGGCGTGCCGTTC
ATGTTGTTCCTCACCCGGGTGCGACATGCGGGCATGGTGACGGTGTTTGCGA
TTATCACGGCCGGACTGCTCGCACTGACCGGGCACCCCCCGATCTGCTTCGT
GATCACAGTTGCGTGCGCGTTGGTGGCCGAAGTCGTCCTGTGGCTGGACGC

TATCGCTCCCGCACCATGGGTGTACTGGCGTACGCAATCTACGCGGCGTGGT
ACATCGGGCCGCTGCTGCCCATCTTCTACGCTCGCGATGAATATTTCTCCAG
TCCCGGCATGGCACAGATGGGTCCGCGCTACCTCGAAGAGATGGAACGGTTG
TTGTCGCCAGCCGTGCTAATCGCATTCGACCTGTCCACGGTGGTATTCGGGC
TGATCGGCGGACTGCTCGGAGTAAGGTTGCTGCGCAAGCATTTTCAGAGGGC
CGGCCTAGCTTGACGCGGACAACGACTCGGGTGACCCAACTCGACCCTCGAA
CCAAAACCGTGCTGGTGCTGGCCTCAAGCATCGCCGTGATGGCCCCCGGTGG
CGAGGTGTTCGTCCCAGCCGCCGTCATCGTTGGGATGCTGCTGGCGGTTGCC
GAgCAGGCGTGGGTGCGGCTGCCATTCTTCCCTCGGCTGCCGGGGCCACGGC
CGCAGTCGCTTACCTACTACCACAAGCTATTCCACATCCGATAATCGGCGCA
ATCGGTACCGTAGCCGCCTACCTGCTACGGCTCATCGCCGTCGGCGCAATCG
TCATTCATCTGGTGAACACCACGACACCGAGCGAATTCACCGCTGCCCTGCG
CGCTACCCACATTCCCCGTGCGATCACGGTCTCCGGTTCCGTCATGCTGCGA
TTCCTGCCAACCATCGTTGGTGAAGCACGTGCCGTCAGCGACGCCATGCGAT
TGCGTGGGATCGGCGGACATACGGAATGCTGCGCCATCCCGTTTGCACCAT
CGAGTACTTCACCGTTCCCCTTATAGCGTCGAGCCTTCGTGTCGCAGAAGAT
CTTTCAGCAACCGCGCTACTGCGTGGTCTTGGCTCTGCAGCACGGCCGACAA
CGATGTACCCCCCGCGTTTCGGTAAAGCGGATGCGCTAATCGGTTGCATCGT
CAGCGCACTGACCGTCACCACCGTGCTGTGGCCGGTGAAACCGTGATTCGCA
TCGACGGCGTACGTTGGCAATACGCCGGAACCGACGCTGCCGTTTTGGACGG
AGTGGACTTGCACATACGCCGTGGCGAGACCGTTCTGCTCTGCGGGGCCAGC
GGATCGGGCAAGAGCAGCGTCCTGCGGCTGATGAATGGCCTTATCCCACATT
TCCACCAGGGCAGCCTCGACGGTAGCGTACACATTGACGGTACATCGGTCGC
CGAATTGTCTCTGGAAAGGGTTGGTCGGCTGACAGGTACCGTATTGCAACAC
CCGCGCCGACAATTCTTCACCGCGGCTGTCGACACCGAATTGGCTTTCACTC
TGGAAAATTTCGGGACACCGCCCGAGCAGATCCGAAACCGTGTCGGCTCCGT
GATCACCGAATACGGGCTCGCAGAACTCACCGGCCACCGATTGGCGGAGTTG
TCCGGCGGCCAGCAGCAACAGATCGCCTGCGCGGCCGCCGCCACCCACGGTC
CGCCGCTGCTGTTGTTCGACGAGCCAACGGCAAACCTGGCTGCGGACGCGAT
CGAGCGTTTCACAGCAACGCTTGCGAGGCTTCGCTCACTGGAACCACGATC
GTCATTGCCGAACACCGGCTGCACTACCTACGCGAAATCGCGGACCGAATCG
TCCTGCTACGCAACGGACGTATCGCCGCCGAATGGTCGCGTAAGCAATTCGC
GCGTCTTGACGATGCGGCGTTGAACGCAGAGGGCCTGCGCAGCAACAATTCT
CCAGTGCGCAATCACATACCCCCGGCGTGCGCATACGGCGCAAGCGTTGCGG
GCACACCATCGGGCACGGCTGCCCCAGCTTCGTCACCGTCGGAGGTGGTGCT

GCGAGGTATTCGTTGTTGCTTCCGCGGCCACCGCGTACTCGACATCGAAGAG
GCGCGGTTTCCGGCTGCAACGGTCACCGCCATCACCGGCCCGAACGGCGCGG
GCAAAAGTACCCTGGCCAGGGTTCTCGTCGGGCTGCAACGTCATGACGGCGA
GGTGTCGTTTGGCGGAAGCCGGATATCTCACAGCCGTCGGCAGCGAATGTCA
CCGATCGTGATGCAGGATGTGCAGCGGCAGCTGTTCACCGAGTCTGTCCGTG
CCGAACTACGGCTCGGTGCACCCCCGGCGGCAGCAGGTGTTGCATCCACCCT
GTTAAGAGACCTGGGGCTGGAAGAGTTCGCCGATCGGCACCCATTGTCACTT
TCGGGAGGACAGCAGCAACGTCTGGTGGTCGCCGCCGCACGGCTGAGCAATC
GCAAGATCATGGTTTTCGACGAACCGAGCTCGGGTGTCGACCGGCGCCACCT
CCGCTCGATCACCAATGTCATGCGTGACGTCGCGGCCCAAGGAGTAGTCGTT
ATCTTGATCAGTCACGATCAGGAGCTGTTGACTCTCGCGGCTGACCAGGAAC
TGCGCATGCGGGTTGCCGACACTCTCAATGCGAGATCCCGTAGGAAGGCGGC
CGGCGAAAATGCTTGCCTTGAAACGCTATCCGACTAGCAGACCCCCGCTCGC
ATGTTACAGCGACAAAGCACTCAGATTGTTCTGAGCTACAACAGTGACCGGG
TGCGCGGGAAGTCTGTTGGAGATCGTCTGCCTTGCCCGGGTCGCCCCCTATG
GATGCGTTACCGTCAACGCAGACGTCGAGCCAGCAGCACATCGTCGTGCGTG
TGCTGCAGTTCTGGCCTGGCGGCTGTCTTGCGGCGCTGCCGTCTGTCGACCC
GGACATGCCAATCGCCGTCAAGCAACGTGGCAATGTCGTCATGGCTGAGGTA
GTCGGCTGGGTCGAACCCGTAGGATTTTGCCTTTTCCGCGTCAACGTCGGCA
TGGTGTACGACAAGGAGTGTCCCTCCGGGAGCGACAGCTGCAAGCAGGGACT
CCTCAGCGGCGCGCCCCGACGAGTGACGCAACGGCCCGGATAAAACGCAATA
ACCAGGTCGTAGCCGCCAACTCGAAGCGGTGCGTTTTCGAATGCGGCACCGC
ACCCAGTTAACCCTGACCCCTTGCTTGTCGACCGGGAGCCCGTTGCGCGTGC
TTAATGCGAACTCGTGACACGTCCAACGCCGTCACGTCCCATTCACGACTGG
CCAGCCAAATCGCTTCAGCGCCTACGCCGCACCCGATGTCGAGTGCCCTTCC
CGGCGCGAACTTGCTTACCTCAGTTATCAGCACGGCATTGGGTTCTGCATCG
CGCATCTCTGTCGTCAAACCGGCGTAGCGCTCATCCCACTCAGCAGCCAGGG
CGTTGGTGTCGTCCGGTGAAGCTGTCATGCATACCAGCATGGCCCAGGTACC
GAAGACCCACAATCGCGATCGCCAAATCGGCAAACTGCCTCGACATCGCTCG
GCAAGTTCGATCGTTCAGCAAAGTCGCTTTCGGGCGGACATCCGAATGTCA
ACAACTTTTGAAAGCTGACCCCGTACCAACTTCTGAATCTTGCCCCCGGGTG
CGGTCCTGCCCGCGAGAGCATTTGCTTTGGTGACCGTCGCTCTGCTCTGCGG
GCAGACTCGATACCCGCAACCCCTAACGGTCAGATCCGTTGTGCGGCACGCA
TAACCGATTGATC.

2. *M*. *paratuberculosis* specific ABC-transporter operon according to claim 1,
that comprises six open reading frames mptA, mptB, mptC, mptD, mptE and mptF.

3. *M*. *paratuberculosis* specific ABC-transporter operon according to claims 1 and 2,
that encodes five proteins.

4. *M*. *paratuberculosis* specific ABC-transporter operon according to claims 1 to 3,
that encodes the proteins MptAB, MptC, MptD, MptE and MptF.

5. Protein MptAB with the sequence (SEQ ID NO 2)

1 VRFDVIWARA DPLSSHANTP VMKIIFITDG DPTALRPLMK VIFNNPDVTE

51 VTLVAGCAPW RSASDARLTA DRMPCPRFGR GGCDALRSWQ CSGFHSLHAG

101 TPYARAPPER PEANSAARRS FQLSGRRHSH RINRRSPRGL ALLSVSEGTA

151 PYPLIHPLRG GMLSITIFII GSRWLGLSAE GLSMDPNTPS QVGPQSPVFG

201 AARATLIGCA LLTALSALAG MAPLIAVVEV SRRLLAGDTA VWAIIAAALV

251 ALAVKQLGTF CAGVLTHLAD IQVSFRIRRD LLVKLSKLPL GWFTDRNSGI

301 VKKTVEDDVS GPAPADRPFH RGTHRGDSSP ISRLDLPVCC.

6. Protein according to claim 5,
that is an ABC-transporter protein.

7. Protein MptC with the sequence (SEQ ID NO 3)

1 MSTMIRYLLE LLDERGRRHL RVMLALQAAQ GILQGLGFLF VVPLMGVLIH

51 KPVDTHALWC WIVAIAVAVV AHHGLLAWST SLGYLVGTDV LTSFHTRIGN

101 HLATLPIGWF RTDRTGPIGR LLSKGTMDVA DLPAHLLRHV IVGVAAPATM

151 IIGSYVLDWR VGLAFTAGAL LCALTLRLLM VVIHRNDDQY DHDIGVTASR

201 IVEYARQQPT LRAYGVLNRP GLGTLEESLV RQQTSQSRLT IRGAWALIGF

251 FGTVQLVVTA IIALTVALAV SGSLALSTMV GLLIITLRMI DPISQLGDLA

301 GHVQVNADAI RRVRELLTVP PLPEPTHPGQ ATGADIELQG VRFAYDGGQP

351 TIDGIDAVFP QGSLTAVVGP SGAGKSTLLK LIGRFFDVDE GSITIGGCDI

401 RQLGTAGVSH LTAQVFQDVY LFEGTIADNL RMADPQATDD DLRHVATISR

451 LDEVVDRLPN GWETRVGEAG STLSGGEKQR VSIARALLKD APVVLLDEAT

501 AALDAANETA VSDAIHELAR DRTVIVVAHR LSTVAAADQI LVLDGGRITE

551 RGRHDTLVGA GGTYARFWEE RTRARGWQLR QIAPTPELQA PRP.

8. Protein according to claim 7,
with a molecular mass of 64 kDa.

9. Protein according to claims 7 and 8,
that is an ABC-transporter protein.

10. Protein MptD with the sequence (SEQ ID NO 4)

1 MTATSSTTQS SRRIDVRMSA RDLINIGVFG ALYIATVFAI NVFAFINPLV

51 MLVALAVSMI AGGVPFMLFL TRVRHAGMVT VFAIITAGLL ALTGHPPICF

101 VITVACALVA EVVLWLGRYR SRTMGVLAYA IYAAWYIGPL LPIFYARDEY

151 FSSPGMAQMG PRYLEEMERL LSPAVLIAFD LSTVVFGLIG GLLGVRLLRK

201 HFQRAGLA.

11. Protein according to claim 10,
that is a transmembrane protein.

12. Protein MptE with the sequence (SEQ ID NO 5)

1   MAPGGEVFVP AAVIVGMLLA VAEQAWVRAA ILPSAAGATA AVAYLLPQAI

51   PHPIIGAIGT VAAYLLRLIA VGAIVIHLVN TTTPSEFTAA LRATHIPRAI

101  TVSGSVMLRF LPTIVGEARA VSDAMRLRGI GGTYGMLRHP VCTIEYFTVP

151  LIASSLRVAE DLSATALLRG LGSAARPTTM YPPRFGKADA LIGCIVSALT

201  VTTVLWPVKP.

13. Protein MptF with the sequence (SEQ ID NO 6)

1 VIRIDGVRWQ YAGTDAAVLD GVDLHIRRGE TVLLCGASGS GKSSVLRLMN

51 GLIPHFHQGS LDGSVHIDGT SVAELSLERV GRLTGTVLQH PRRQFFTAAV

101 DTELAFTLEN FGTPPEQIRN RVGSVITEYG LAELTGHRLA ELSGGQQQQI

151 ACAAAATHGP PLLLFDEPTA NLAADAIERF TATLARLRSL GTTIVIAEHR

201 LHYLREIADR IVLLRNGRIA AEWSRKQFAR LDDAALNAEG LRSNNSPVRN

251 HIPPACAYGA SVAGTPSGTA APASSPSEVV LRGIRCCFRG HRVLDIEEAR

301 FPAATVTAIT GPNGAGKSTL ARVLVGLQRH DGEVSFGGSR ISHSRRQRMS

351 PIVMQDVQRQ LFTESVRAEL RLGAPPAAAG VASTLLRDLG LEEFADRHPL

401 SLSGGQQQRL VVAAARLSNR KIMVFDEPSS GVDRRHLRSI TNVMRDVAAQ

451 GVVVILISHD QELLTLAADQ ELRMRVADTL NARSRRKAAG ENACLETLSD.

14. Transformants of M. smegmatis mc²155 containing the *M*. *paratuberculosis* specific ABC-transporter operon according to claim 1,
**characterized in that** the integrative and kanamycinselectable shuttle vector pMV361 was used.

15. Protein MptC, **characterized in that** it was expressed in *M*. *smegmatis.*

16. Use of the proteins MptAB and/or MptC and/or MptD and/or MptE and/or MptF for the isolation of peptides that specifically bind to said proteins.

17. Antibody against the *M*. *paratuberculosis* specific ABC-transporter encoded by the sequence (SEQ ID NO 1) according to claim 1.

18. Antibody against the proteins MptAB and/or MptC and/or MptD and/or MptE and/or MptF.

19. Use of the proteins MptAB and/or MptC and/or MptD and/or MptE and/or MptF for manufacturing an antiserum.

20. Method for the preparation of antiserum against at least one of the proteins MptAB or MptC or MptD or MptE or MptF, **characterized in that** it is raised in an animal.

21. Method according claim 20,
**characterized in that** the antiserum was raised in a rabbit.

22. Method according to claims 20 and 21,
**characterized that** an initial injection of protein MptC was followed by two booster injections of recombinant fusion protein, emulsified in adjuvant.

23. A therapeutical composition against paratuberculosis and/or Crohn's disease, **characterized in that** it contains protein MptAB or protein MptC or protein MptD or protein MptE or protein MptF or at least one fragment of at least one of said proteins or a mixture thereof.

24. A therapeutical composition according to claim 23,
**characterized in that** it is a vaccine.

25. Vaccine according to claim 24,
**characterized in that** it further contains pharmaceutically acceptable agents, carriers, solvents and/or adjuvants.

26. A method for inducing an immunological response in an animal or human in which the *M*. *paratuberculosis* specific ABC-transporter operon or at least one of the proteins MptAB or MptC or MptD or MptE or MptF or at least one fragment of at least one of said proteins or a mixture thereof, are used to produce an antibody to protect said animal or human from paratuberculosis and/or Crohn's disease.
